# NOUVEAU FASCICULE DE BREVET EUROPEEN

(11) **EP 1 049 447 B2**
(45) Date de publication et mention de la décision concernant l'opposition: **03.03.2010**
(45) Mention de la délivrance du brevet: 01.06.2005
(21) Numéro de dépôt: 99934810.5
(22) Date de dépôt: 29.07.1999
(51) Int. Cl.: A61Q 5/06, A61K 8/49, A61K 8/894, A61K 8/898

(54) **COMPOSITION DE TEINTURE POUR FIBRES KERATINIQUES AVEC UN COLORANT DIRECT CATIONIQUE ET UNE SILICONE**
FÄRBEMITTEL FÜR KERATINFASERN, DAS EIN KATIONISCHER FARBSTOFFE UND EINE SILICONVERBINDUNG ENTHÄLT
DYEING COMPOSITION FOR KERATINOUS FIBRES WITH A CATIONIC DIRECT COLOURING AGENT AND A SILICONE

(30) Priorité: 26.08.1998 FR 9810724
(43) Date de publication de la demande: 08.11.2000
(73) Titulaire: L'Oréal, 75008 Paris (FR)
(72) Inventeur: RONDEAU, Christine, F-78500 Sartrouville (FR); LANG, Gérard, F-95390 Saint Prix (FR); COTTERET, Jean, F-78480 Verneuil/Seine (FR)
(74) Mandataire: Miszputen, Laurent
(86) Numéro de dépôt international: PCT/FR1999/001876
(87) Numéro de publication internationale: WO 2000/012057

(56) Documents cités:
- EP-A- 0 850 637
- EP-A- 0 850 638
- WO-A-95/01772
- WO-A-95/15144
- WO-A-99/20234
- WO-A-99/20235
- FR-A- 2 140 205
- FR-A- 2 189 006
- FR-A- 2 282 860
- FR-A- 2 285 851
- GB-A- 2 168 082
- GB-A- 2 173 515
- US-A- 5 143 518

## Description

L'invention concerne une composition de teinture pour fibres kératiniques, en particulier pour fibres kératiniques humaines telles que les cheveux, comprenant, dans un milieu approprié pour la teinture, au moins un colorant direct cationique de formule donnée, et au moins une silicone particulière.

L'invention a également pour objets les procédés et dispositifs de teinture mettant en oeuvre ladite composition.

Dans le domaine capillaire, on peut distinguer deux types de coloration.

Le premier est la coloration semi-permanente ou temporaire, ou coloration directe, qui fait appel à des colorants capables d'apporter à la coloration naturelle des cheveux, une modification de couleur plus ou moins marquée résistant éventuellement à plusieurs shampooings. Ces colorants sont appelés colorants directs; ils peuvent être mis en oeuvre avec ou sans agent oxydant. En présence d'oxydant, le but est d'obtenir une coloration éclaircissante. La coloration éclaircissante est mise en oeuvre en appliquant sur les cheveux le mélange extemporané d'un colorant direct et d'un oxydant et permet notamment d'obtenir, par éclaircissement de la mélanine des cheveux, un effet avantageux tel qu'une couleur unie dans le cas des cheveux gris ou de faire ressortir la couleur dans le cas de cheveux naturellement pigmentés.
Le deuxième est la coloration permanente ou coloration d'oxydation. Celle-ci est réalisée avec des colorants dits "d'oxydation" comprenant les précurseurs de coloration d'oxydation et les coupleurs. Les précurseurs de coloration d'oxydation, appelés couramment "bases d'oxydation", sont des composés initialement incolores ou faiblement colorés qui développent leur pouvoir tinctorial au sein du cheveu en présence d'agents oxydants ajoutés au moment de l'emploi, en conduisant à la formation de composés colorés et colorants. La formation de ces composés colorés et colorants résulte, soit d'une condensation oxydative des "bases d'oxydation" sur elles-mêmes, soit d'une condensation oxydative des "bases d'oxydation" sur des composés modificateurs de coloration appelés couramment "coupleurs" et généralement présents dans les compositions tinctoriales utilisées en teinture d'oxydation.
Pour varier les nuances obtenues avec lesdits colorants d'oxydation, ou les enrichir de reflets, Il arrive qu'on leur ajoute des colorants directs.

Parmi les colorants directs cationiques disponibles dans le domaine de la teinture des fibres kératiniques notamment humaines, on connaît déjà les composés dont la structure est développée dans le texte qui va suivre; néanmoins, ces colorants conduisent à des colorations qui présentent des caractéristiques encore insuffisantes sur le plan de la puissance, de l'homogénéité de la couleur répartie le long de la fibre, on dit alors que la coloration est trop sélective, et sur le plan de la tenacité, en terme de résistance aux diverses agressions que peuvent subir les cheveux (lumière, intempéries,shampooings).

Or, après d'importantes recherches menées sur la question, la demanderesse vient maintenant de découvrir qu'il est possible d'obtenir de nouvelles compositions pour la teinture des fibres kératiniques capables de conduire à des colorations puissantes et néanmoins peu sélectives et résistant bien aux diverses agressions que peuvent subir les cheveux, en associant au moins une silicone particulière à au moins un colorant direct cationique connu de l'art antérieur et de formules respectivement définies ci-après.

Cette découverte est à la base de la présente invention.

La présente invention a donc pour premier objet une composition pour la teinture des fibres kératiniques et en particulier des fibres kératiniques humaines telles que les cheveux, renfermant dans un milieu approprié pour la teinture, (i)au moins un colorant direct cationique dont la structure répond aux formules (I) à (IV) définies ci-après, caractérisée par le fait qu'elle contient en outre (ii)au moins une silicone particulière.

(i) Le colorant direct cationique utilisable selon la présente invention est un composé choisi parmi ceux de formules (I), ou, (III) suivantes :
**a) les composés de formule (I) suivante :** 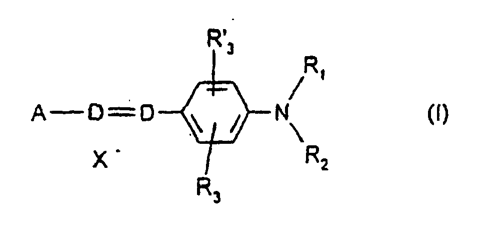 dans laquelle :
   D représente un atome d'azote ou le groupement -CH,
   R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
   R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
   X ⁻ représente un anion de préférence choisi parmi le chlorure, le méthyl sulfate et l'acétate,
   A représente un groupement choisi par les structures A1 à A18 suivantes : 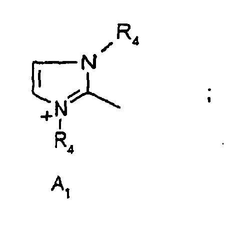 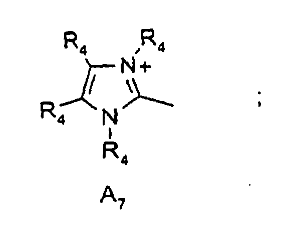 et 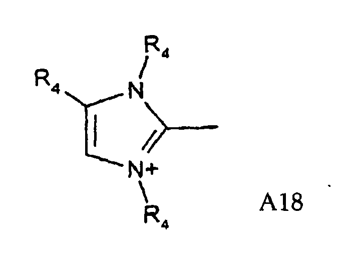
   dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**c) les composés de formule (III) suivante :** 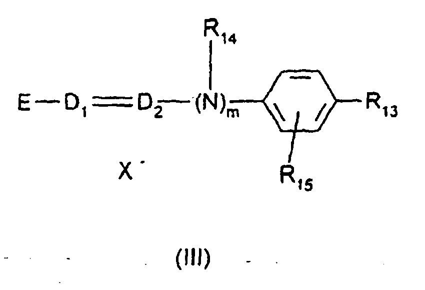 dans lesquelles :
   R₁₃ représente un atome d'hydrogène, un radical alcoxy en C₁-C₄, un atome d'halogène tel que le brome, le chlore, l'iode ou le fluor ou un radical amino,
   R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
   R₁₅ représente un atome d'hydrogène ou d'halogène tel que le brome, le chlore, l'iode ou le fluor,
   D₁ et D₂, identiques ou différents, représentent un atome d'azote ou le groupement -CH,
   m = 0 ou 1,
   étant entendu que lorsque R₁₃ représente un groupement amino non substitué, alors D₁ et D₂ représentent simultanément un groupement -CH et m = 0,
   X ⁻ représente un anion de préférence choisi parmi le chlorure, le méthyl sulfate et l'acétate,
   E représente un groupement choisi par les structures E1 à E8 suivantes : 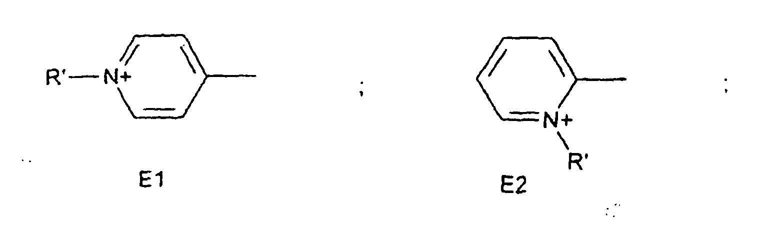 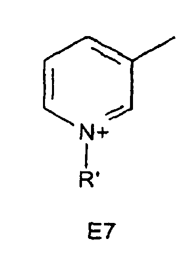 dans lesquelles R' représente un radical alkyle en C₁-C₄.

Dans les structures (I) et (III) définies ci-dessus le groupement alkyle ou alcoxy en C₁-C₄ désigne de préférence méthyle, éthyle, butyle, méthoxy, éthoxy.

Les colorants directs cationiques de formules (I) et (III) utilisable dans les compositions tinctoriales conformes à l'invention, sont des composés connus et sont décrits par exemple dans les demandes de brevets WO 95/01772, WO 95/15144 et EP-A-0 714 954.

Parmi les colorants directs cationiques de formule (I) utilisables dans les compositions tinctoriales conformes à l'invention, on peut plus particulièrement citer les composés répondant aux structures suivantes :

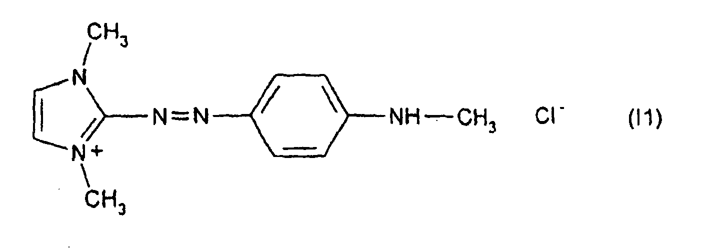

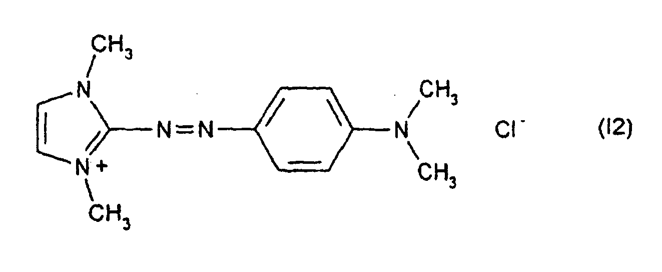

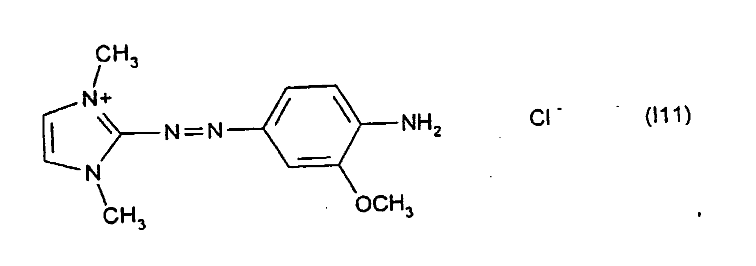

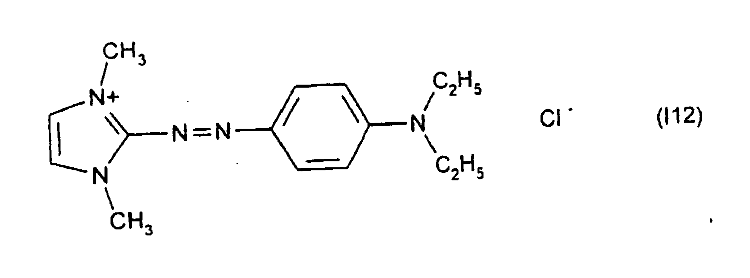

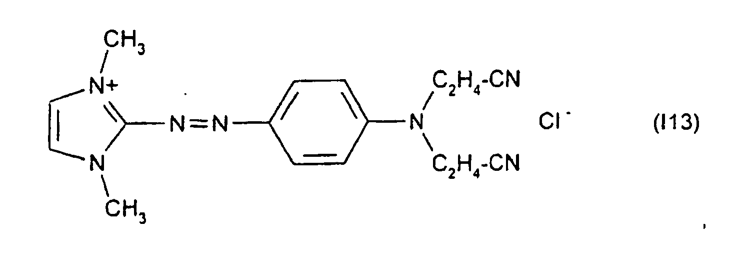

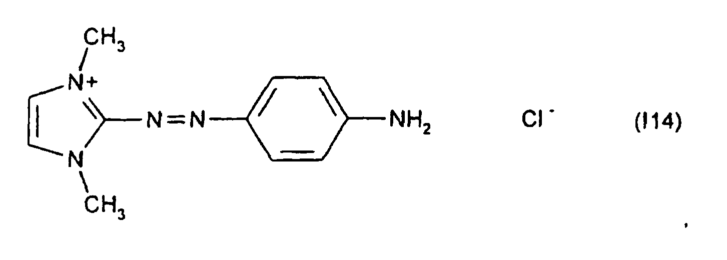

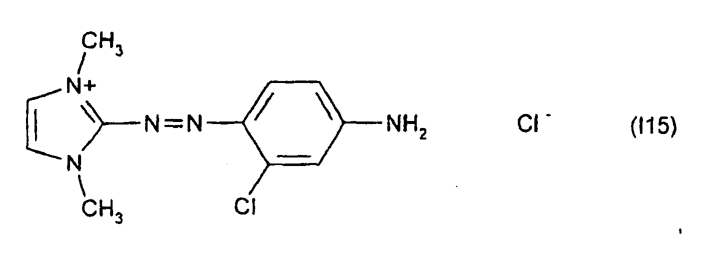

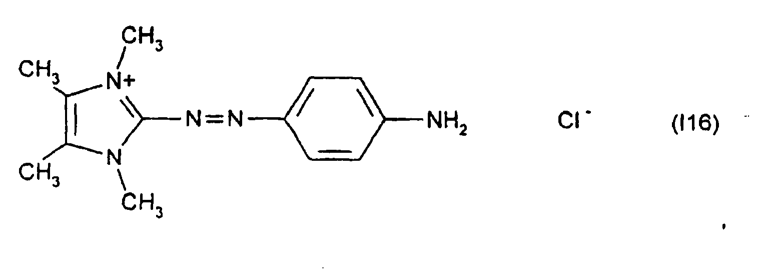

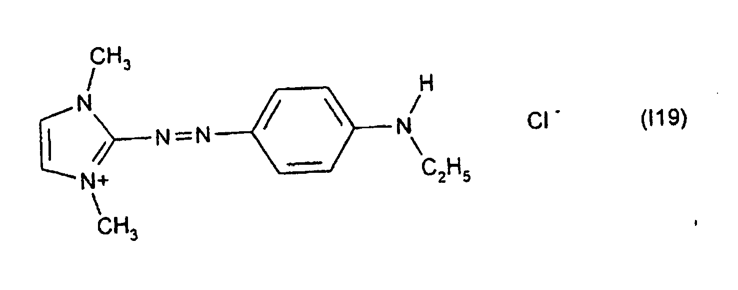

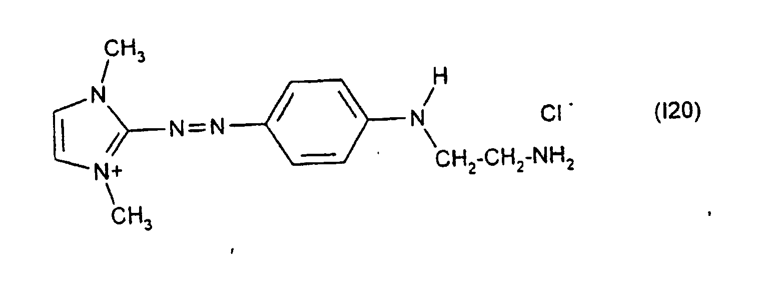

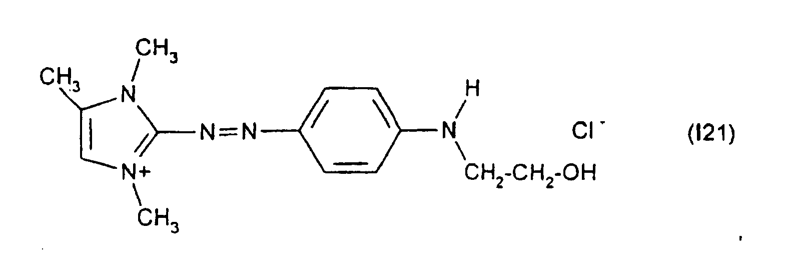

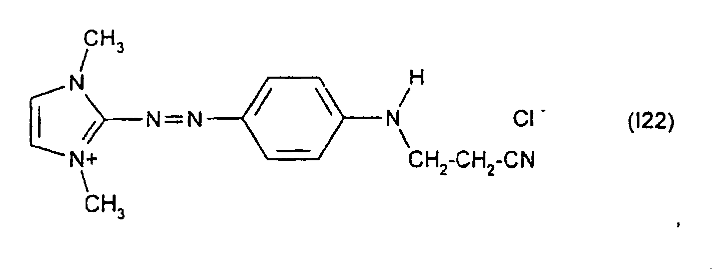

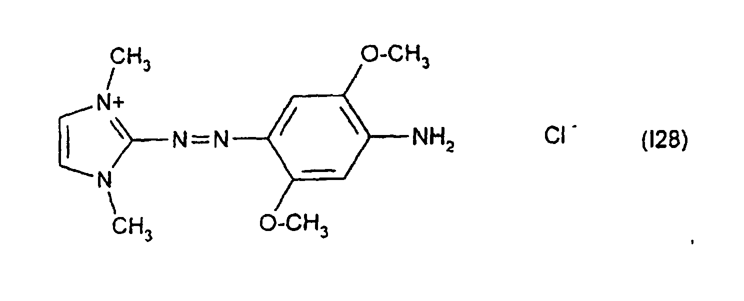

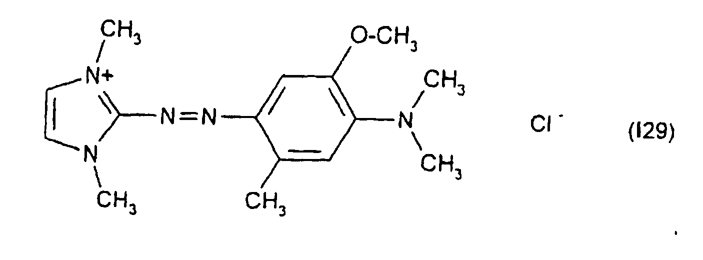

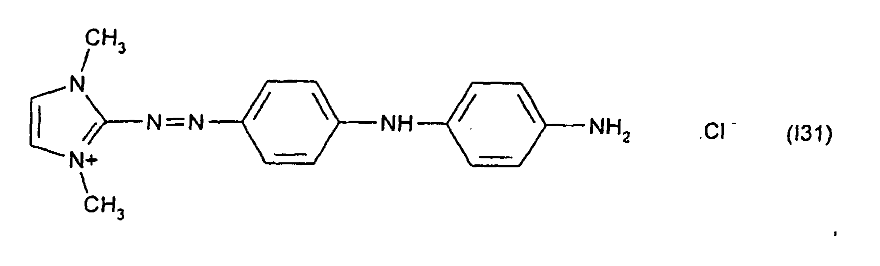

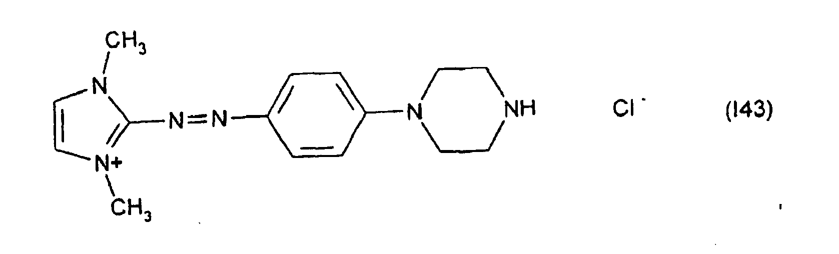

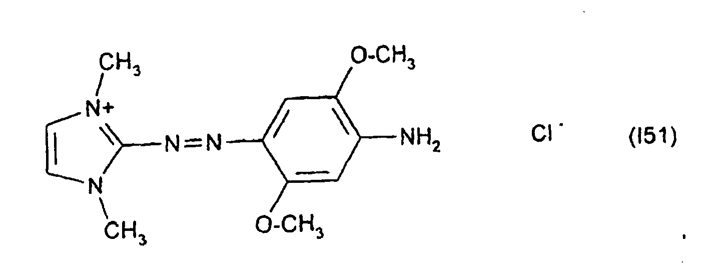

Parmi les composés de structures décrits ci-dessus, on préfère tout particulièrement les composés répondant aux structures (I1), (I2), (I14) et (I31).

Parmi les colorants directs cationiques de formule (III) utilisables dans les compositions tinctoriales conformes à l'invention, on peut plus particulièrement citer les composés répondant aux structures suivantes :

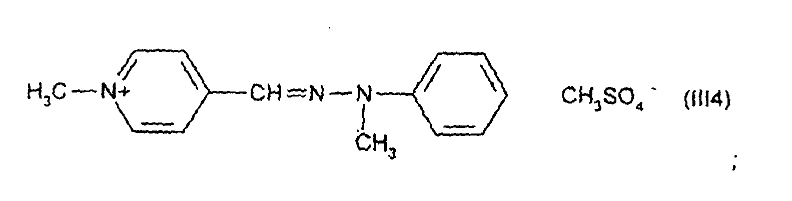

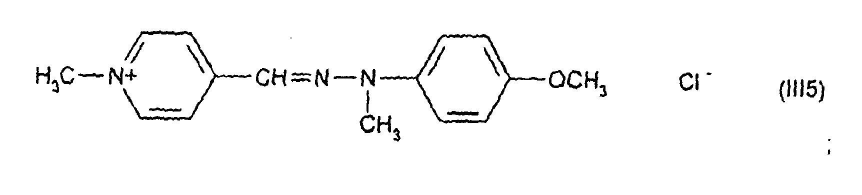

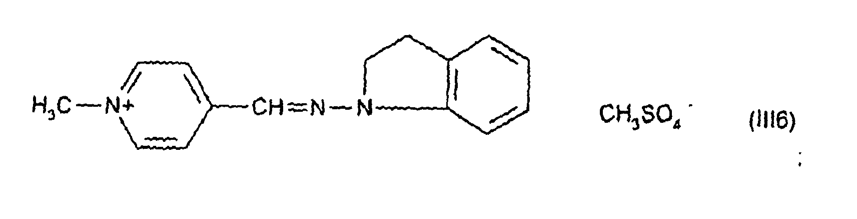

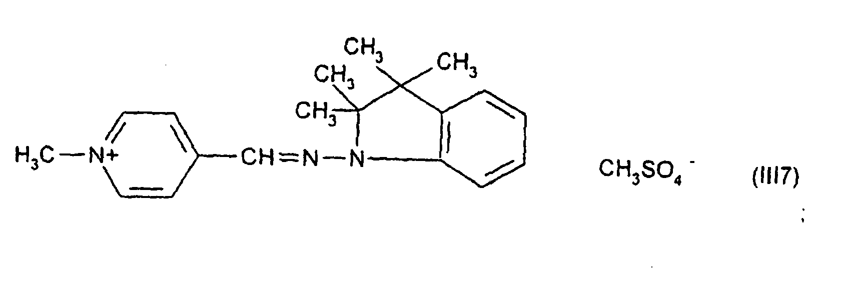

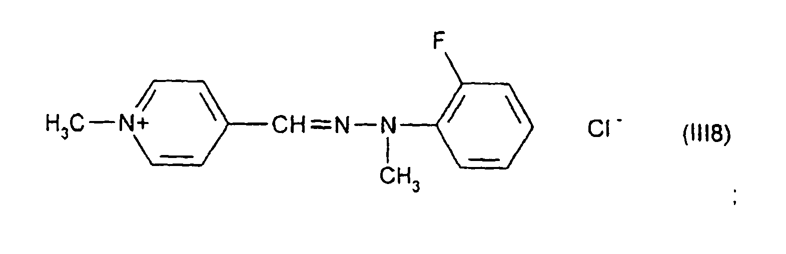

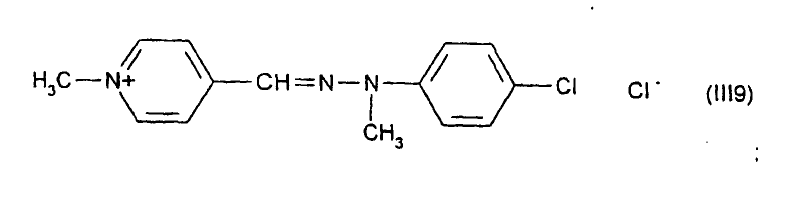

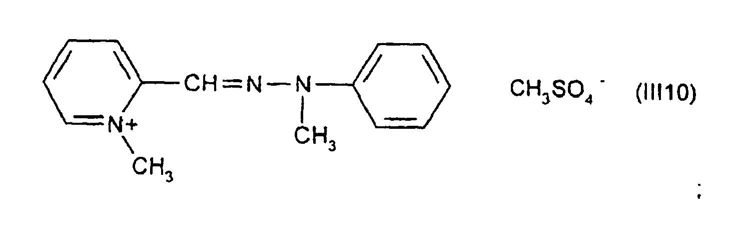

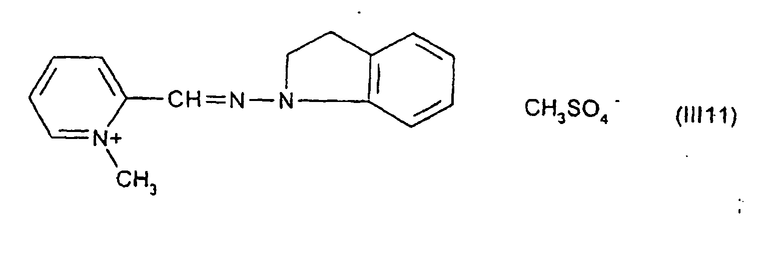

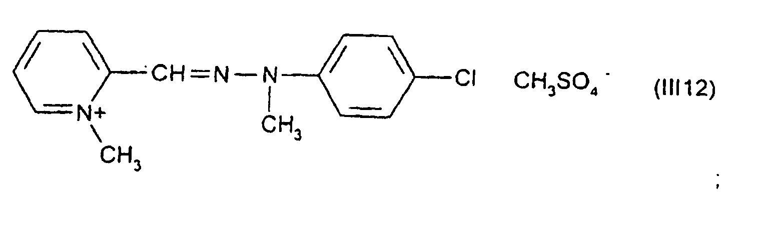

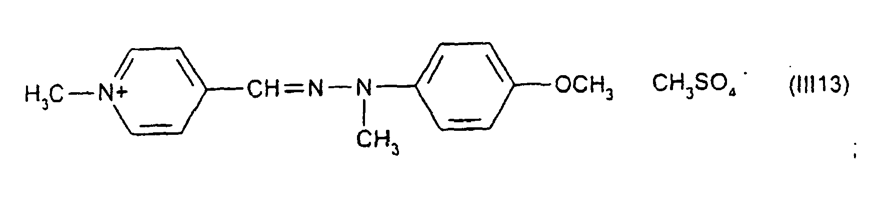

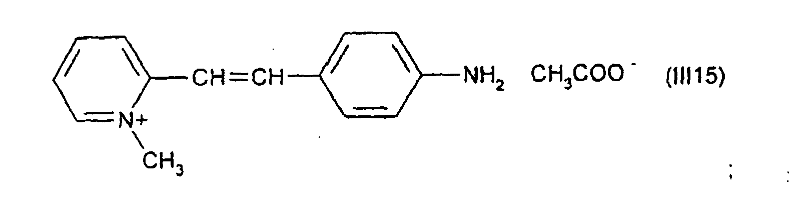

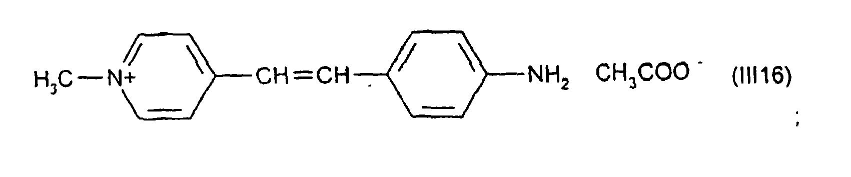

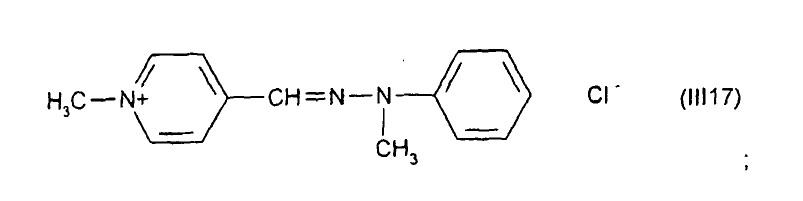

Parmi les composés particuliers de structures décrits ci-dessus, on préfère tout particulièrement les composés répondant aux structures (III4), (III5) et (III13).

Le ou les colorants directs cationiques utilisés selon l'invention, représentent de préférence de 0,001 à 10 % en poids environ du poids total de la composition tinctoriale et encore plus préférentiellement de 0,005 à 5 % en poids environ de ce poids.

(ii) Les silicones utilisables selon la présente invention sont choisies dans le groupe constitué par :
(ii)**₁** les silicones aminées,
(ii)**₂** les silicones polyoxyalkylénées,
(ii)**₃** les gommes et résines de silicones.

Dans toute la présente invention, on entend désigner par silicone, en conformité avec l'acceptation générale, tous polymères ou oligomères organosiliciés à structure linéaire ou cyclique, ramifiée ou réticulée, de poids moléculaire variable, obtenus par polymérisation et/ou polycondensation de silanes convenablement fonctionnarisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués, étant directement liés par l'intermédiaire d'un atome de carbone sur lesdits atomes de silicium. Les radicaux hydrocarbonés les plus courants sont les radicaux alkyles, notamment en C₁-C₁₀, et en particulier méthyle, les radicaux fluoroalkyles, les radicaux aryles et en particulier phényle.

Selon l'invention, on désigne par silicone aminée toute silicone comportant au moins une amine primaire, secondaire, tertiaire ou un groupement ammonium quaternaire.

Selon l'invention également, on désigne par silicone polyoxyalkylénée toute silicone comportant au moins un groupement oxyalkyléné de type (-C**ₓ**H_{2**x**}O-)**ₐ** dans lequel **x** peut varier de 2 à 6, et a est supérieur ou égal à 2.

Conformément à l'invention, les silicones aminées (ii)**₁** sont choisies parmi :
**(ii)₁₍a)** les composés dénommés dans le dictionnaire CTFA, "amodiméthicone" et répondant à la formule (V) suivante : 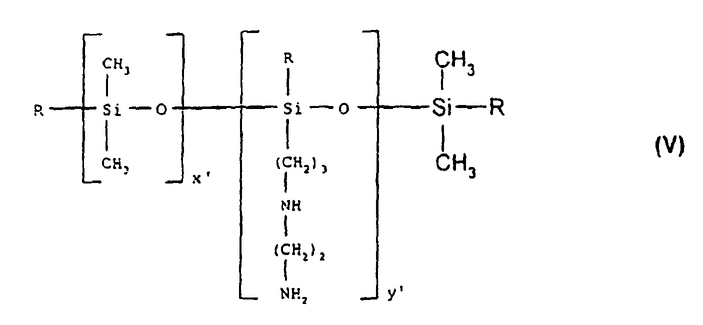 dans laquelle R désigne le radical CH₃ ou OH, et
   x' et y' sont des nombres entiers dépendant du poids moléculaire, généralement tels que ledit poids moléculaire en nombre est compris entre 5 000 et 500 000 environ ;
**(ii)₁(b)** les composés répondant à la formule (VI) suivante :

   (R¹)ₐ (T)₃₋ₐ -Si[OSi(T)₂]ₙ -[OSi(T)_{b}(R¹)_{2-b}]ₘ -OSi(T)₃₋ₐ - (R¹)ₐ (VI)

   dans laquelle,
   T est un atome d'hydrogène, ou un radical phényle, ou OH, ou alkyle en C₁-C₈, et de préférence méthyle,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R¹ est un radical monovalent de formule -C_{q}H_{2Q}L dans laquelle q est un nombre de 2 à 8 et L est un groupement aminé éventuellement quaternisé choisi parmi les groupements :
   -N(R²)-CH₂-CH₂-N(R²)₂
   -N(R²)₂
   -N^{⊕}(R²)₃ Q⁻
   -N^{⊕}(R²)(H)₂ Q⁻
   -N^{⊕}(R²)₂H Q⁻
   -N(R²)-CH₂-CH₂-N^{⊕}(R²)(H)₂ Q⁻,
   dans lesquels R² peut désigner hydrogène, phényle, benzyle, ou un radical hydrocarboné saturé monovalent, par exemple un radical alkyle ayant de 1 à 20 atomes de carbone et Q⁻ représente un ion halogénure tel que par exemple fluorure, chlorure, bromure ou iodure.
   Un produit correspondant à cette définition est le polymère dénommé dans le dictionnaire CTFA "triméthylsilylamodiméthicone", répondant à la formule (VII) suivante : 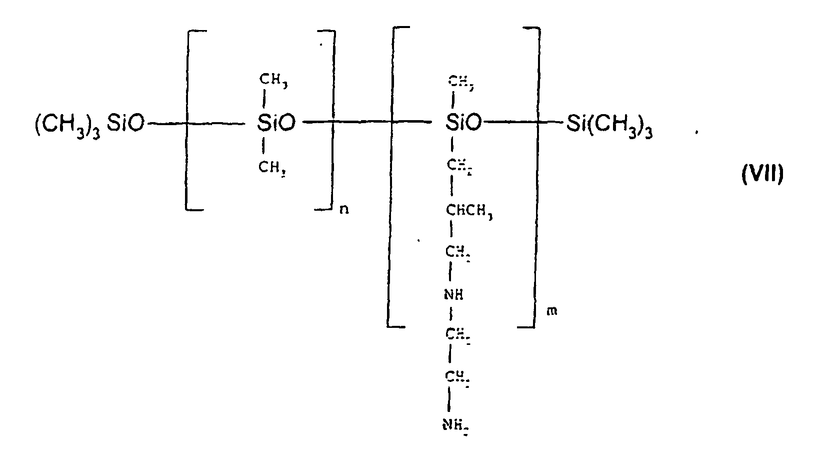 dans laquelle n et m ont les significations données ci-dessus [cf formule (VI)]. De tels composés sont décrits par exemple dans la demande de brevet EP-A-95238; un composé de formule (VII) est par exemple vendu sous la dénomination Q2-8220 par la société OSI.
**(ii)₁(c)** les composés répondant à la formule (VIII) suivante : 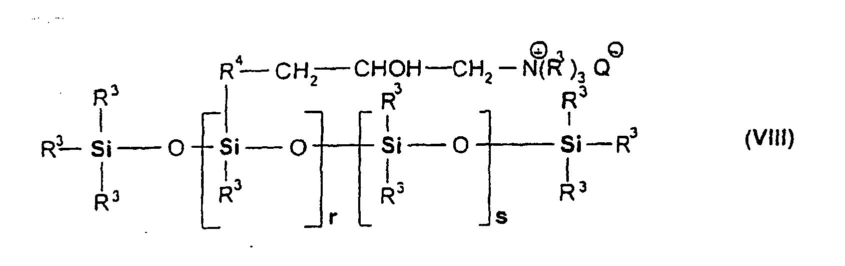 dans laquelle,
   R³ représente un radical hydrocarboné monovalent ayant de 1 à 18 atomes de carbone, et en particulier un radical alkyle en C₁-C₁₈, ou alcényle en C₂-C₁₈, par exemple méthyle ;
   R⁴ représente un radical hydrocarboné divalent, notamment un radical alkylène en C₁-C₁₈ ou un radical alkylèneoxy divalent en C₁-C₁₈, par exemple en C₁-C₈ ;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

De tels composés sont décrits plus particulièrement dans le brevet US-4 185 087.

Un composé entrant dans cette classe est celui vendu par la Société Union Carbide sous la dénomination "Ucar Silicone ALE 56".

Lorsque ces composés sont mis en oeuvre, une forme de réalisation particulièrement intéressante est leur utilisation conjointe avec des agents de surface cationiques et/ou non ioniques. A titre exemple, on peut utiliser le produit vendu sous la dénomination "Emulsion Cationique DC 929" par la Société Dow Corning, qui comprend, outre l'amodiméthicone, un agent de surface cationique comprenant un mélange de produits répondant à la formule : 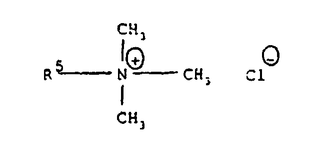 dans laquelle, R⁵ désigne des radicaux alcényle et/ou alcoyle ayant de 14 à 22 atomes de carbone dérivés des acides gras du suif, et connu sous la dénomination CTFA "tallowtrimonium chloride",
en association avec un agent de surface non ionique de formule :

C₉H₁₉-C₆H₄-(OC₂H₄)₁₀-OH,

connu sous la dénomination CTFA "Nonoxynol 10".

On peut également utiliser par exemple le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule :

C₁₃H₂₇-(OC₂H₄)₁₂-OH,

connu sous la dénomination CTFA "tridéceth-12".

Un autre produit commercial utilisable selon l'invention est le produit vendu sous la dénomination "Dow Corning Q2 7224" par la Société Dow Corning, comportant en association le triméthylsilylamodiméthicone de formule (VII) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

Les silicones polyoxyalkylénées **(ii)₂**, selon la présente invention, sont choisies parmi les composés de formules générales (IX), (X), (XI) et (XII) suivantes : 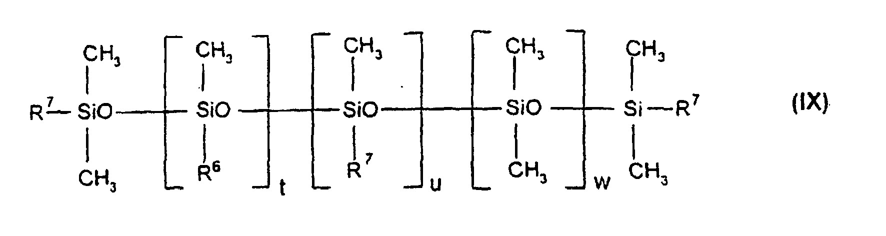 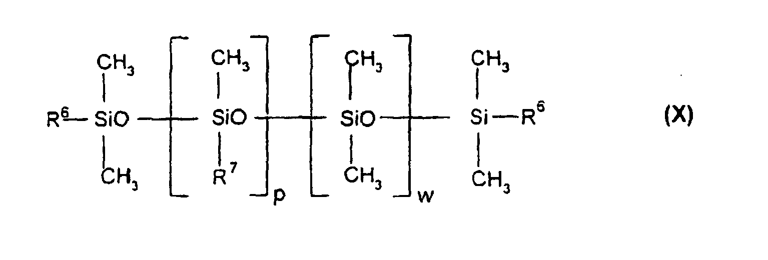 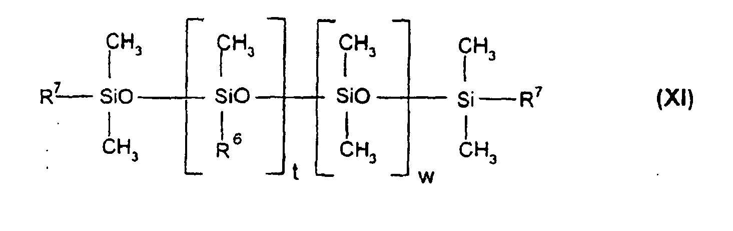 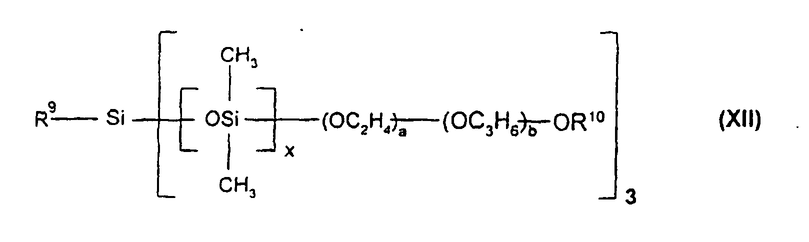 formules (IX), (X), (XI) et (XII) dans lesquelles,
- R⁶, identique ou différent, représente un radical alkyle, linéaire ou ramifié, en C₁-C₃₀ ou phényle,
- R⁷, identique ou différent, représente un radical -C_{C}H_{2c}-O-(C₂H₄O)ₐ(C₃H₆O)_{b}-R⁸ ou un radical -C_{c}H_{2c}-O-(C₄H₈O)ₐ-R⁸,
- R⁹, R¹⁰ identiques ou différents, désignent un radical alkyle, linéaire ou ramifié, en C₂-C₁₂, et de préférence le radical méthyle,
- R⁸, identique ou différent, est choisi parmi un atome d'hydrogène, un radical alkyle, linéaire ou ramifié, en C₁-C₁₂, un radical alcoxy, linéaire ou ramifié, en C₁-C₆, un radical acyle, linéaire ou ramifié, en C₂-C₃₀, un radical hydroxyle, -SO₃M, aminoalcoxy en C₁-C₆ éventuellement substitué sur l'amine, aminoacyle en C₂-C₆ éventuellement substitué sur l'amine, -NHCH₂CH₂COOM, -N(CH₂CH₂COOM)₂, aminoalkyle éventuellement substitué sur l'amine et sur la chaîne alkyle, carboxyacyle en C₂-C₃₀, un groupement phosphono éventuellement substitué par un ou deux radicaux aminoalkyle substitués, -CO(CH₂)_{d}COOM, -COCHR¹¹ (CH₂)_{d}COOM, -NHCO(CH₂)_{d}OH, -NH₃Y, un groupement phosphate,
- M, identique ou différent, désigne un atome d'hydrogène, Na, K, Li, NH₄ ou une amine organique,
- R¹¹ désigne un atome d'hydrogène ou un radical -SO₃M,
- d varie de 1 à 10,
- u varie de 0 à 20,
- w varie de 0 à 500,
- t varie de 0 à 20,
- p varie de 1 à 50,
- a varie de 0 à 50,
- b varie de 0 à 50,
- la somme (a + b) est supérieure ou égale à 2,
- c varie de 0 à 4,
- x varie de 1 à 100,
- Y représente un anion minéral ou organique monovalent tel que halogénure (chlorure, bromure), sulfate, carboxylate (acétate, lactate, citrate),

Ces silicones sont notamment décrites dans les brevets US-A-5 070 171, US-A-5149765, US-A-5093452 et US-A-5091493.

De telles silicones sont par exemple commercialisées par la société RHODIA CHIMIE sous la dénomination MIRASIL DMCO, par la société GOLDSCHMIDT sous les dénominations ABIL WE 09, ABIL EM 90, ABIL B8852, ABIL B8851, ABIL B 8843, ABIL B8842, par la société DOW CORNING sous les dénominations FLUID DC 190, DC 3225 C, Q2-5220, Q25354, Q2-5200, par la société RHONE POULENC sous les dénominations SILBIONE HUILE 70646, RHODORSIL HUILE 10634, par la société GENERAL ELECTRIC sous les dénominations SF1066, SF1188, par la société SWS SILICONES sous la dénomination SILICONE COPOLYMER F 754, par la société AMERCHOL sous la dénomination SILSOFT BEAUTY AID SL, par la société SHIN-ETSU sous la dénomination KF 351, par la société WACKER sous la dénomination BELSIL DMC 6038, par la société SILTECH sous les dénominations SILWAX WD-C, SILWAX WD-B, SILWAX WD-IS, SILWAX WSL, SILWAX DCA 100, SILTECH AMINE 65, par la société FANNING CORPORATION sous les dénominations FANCORSIL SLA, FANCORSIL LIM1, par la société PHOENIX sous la dénomination PECOSIL.

De préférence, selon la présente invention, on utilise les silicones polyoxyalkylénées répondant aux formules générales (X) ou (XI). Plus particulièrement, ces formules répondent à au moins une des, et de préférence, toutes les, conditions suivantes :
- c est égal à 2 ou 3;
- R⁶ désigne le radical méthyle;
- R⁸ représente un radical méthyle, un radical acyle en C₁₂-C₂₂, -CO(CH₂)_{d}COOM;
- a varie de 2 à 25 et plus particulièrement de 2 à 15.
- b est égal à 0.
- w varie de 0 à 100.
- p varie de 1 à 20.

Les silicones polyoxyalkylénées selon l'invention peuvent également être choisies parmi les composés de formule (XIII) suivante :

([Z(R¹²SiO)_{q} R¹³SiZO] [(CₙH₂ₙO)ᵣ])ₛ **(XIII)**

dans laquelle,
- R¹² et R¹³, identiques ou différents, représentent un radical hydrocarboné monovalent,
- n est un nombre entier allant de 2 à 4,
- q est un nombre supérieur ou égal à 4, de préférence compris entre 4 et 200 et encore plus particulièrement entre 4 et 100.
- r est un nombre supérieur ou égal à 4, de préférence compris entre 4 et 200 et encore plus particulièrement entre 5 et 100.
- s est un nombre supérieur ou égal à 4, de préférence compris entre 4 et 1 000 et encore plus particulièrement entre 5 et 300.
- Z représente un groupe organique divalent qui est lié à l'atome de silicium adjacent par une liaison carbone-silicium et à un bloc polyoxyalkylène par un atome d'oxygène,
- le poids moléculaire moyen de chaque bloc siloxane est compris entre environ 400 et environ 10.000, celui de chaque bloc polyoxyalkylène étant compris entre environ 300 et environ 10.000,
- les blocs siloxane représentent de 10% environ à 95% environ en poids du copolymère bloc,
- le poids moléculaire moyen en nombre du copolymère bloc pouvant aller de 2.500 à 1.000.000, et de préférence compris entre 3.000 et 200.000, et encore plus particulièrement entre 6000 et 100000.

R¹² et R¹³ sont préférentiellement choisis parmi le groupe comprenant les radicaux alkyles linéaires ou ramifiés comme par exemple les radicaux méthyle, éthyle, propyle, butyle, pentyle, hexyle, octyle, décyle, dodécyle, les radicaux aryles comme par exemple phényle, naphtyle, les radicaux aralkyls ou alkylaryles comme par exemple benzyle, phényléthyle, les radicaux tolyle, xylyle.

Z est de préférence -R"-, -R"-CO-, -R"-NHCO-, -R"-NH-CO-NH-R'''-, -R"-OCONH-R"'-NHCO-, où R" est un groupe alkylène divalent, linéaire ou ramifié en C1-C6, comme par exemple l'éthylène, le propylène ou le butylène, et R''' est un groupe alkylène divalent ou un groupe arylène divalent comme -C₆H₄-, -C₆H₄-C₆H₄-, -C₆H₄-CH₂-C₆H₄-, -C₆H₄-C(CH₃)₂C₆H₄-.

Encore plus préférentiellement, Z représente un radical alkylène divalent, plus particulièrement le radical -C₃H₆- ou le radical -C₄H₈-, linéaires ou ramifiés.

La préparation des copolymères blocs mis en oeuvre dans le cadre de la présente invention est décrite dans la demande européenne EP-0 492 657 A1, dont l'enseignement est inclus dans la présente description.

De tels produits sont par exemple commercialisés sous la dénomination SILICONE FLUID FZ-2172 par la société OSI.

Les gommes et les résines de silicone **(ii)₃**, selon la présente invention, sont choisies notamment en ce qui concerne :
**(ii)₃(a)** les gommes, parmi les polydiorganosiloxanes de fortes masses moléculaires, comprises entre 200000 et 1000000; on peut citer les gommes suivantes :
   - poly [(diméthylsiloxane)] / (méthylvinylsiloxane)],
   - poly [(diméthylsiloxane( / (diphénylsiloxane)],
   - poly [(diméthylsiloxane) / (phénylméthylsiloxane)],
   - poly [(diméthylsiloxane) / (diphénylsiloxane) / (méthylvinylsiloxane)].

Les résines de silcones sont des systèmes siloxaniques réticulés renfermant les unités R₂SiO_{2/2}, RSiO_{3/2} et SiO_{4/2}, dans lesquelles R représente un groupement hydrocarboné possédant 1 à 6 atomes de carbone ou un groupement phényle. Parmi ces produits, ceux particulièrement préférés sont ceux dans lesquels R désigne un radical alkyle inférieur ou un radical phényle.
On peut citer notamment parmi ces résines, le produit vendu par la société DOW CORNING sous la dénomination DOW CORNING 593, qui est un mélange de triméthylsiloxysilicate et de polydiméthylsiloxane, ou les produits vendus par la société GENERAL ELECTRIC sous les dénominations SILICONE FLUID SS 4230 et SS 4267, qui sont des diméthyl/triméthyl/polysiloxanes.

Parmi toutes les silicones décrites ci-avant, on utilise de préférence selon la présente invention, les silicones aminées référencées (ii)₁(a), c'est-à-dire celles répondant à la formule (V) décrite ci-avant, les silicones aminées référencées (ii)**₁**(b) et de formule (VII) décrite ci-avant, et les silicones polyoxyalkylénées référencées (ii)₂ et de formules respectives (X) et (XI) décrites ci-avant.

La ou les silicones (ii) utilisées selon l'invention, représentent de préférence de 0,01 à 20% en poids environ du poids total de la composition tinctoriale et encore plus préférentiellement de 0,1 à 10% en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; les alcools aromatiques comme l'alcool benzylique, ainsi que les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 2 et 11 environ, et de préférence entre 5 et 10 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (XIV) suivante : 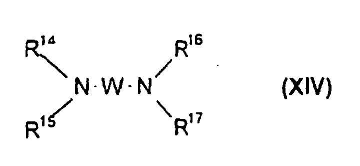 dans laquelle W est un reste propylène éventuellement substitué par un groupement hydroxyle ou un radical alkyle en C₁-C₆ ; R¹⁴, R¹⁵, R¹⁶ et R¹⁷, identiques ou différents, représentent un atome d'hydrogène, un radical alkyle en C₁-C₆ ou hydroxyalkyle en C₁-C₆.

La composition tinctoriale conforme à l'invention peut, en plus du ou des colorants directs cationiques (i) définis précédemment, contenir un ou plusieurs colorants directs additionnels qui peuvent par exemple être choisis parmi les colorants benzéniques nitrés, les colorants anthraquinoniques, les colorants naphtoquinoniques, les colorants triarylméthaniques, les colorants xanthéniques, les colorants azoïques non cationiques.

Lorsqu'elle est destinée à la teinture d'oxydation, la composition tinctoriale conforme à l'invention contient, en plus du ou des colorants directs cationiques (i) une ou plusieurs bases d'oxydation choisie parmi les bases d'oxydation classiquement utilisées pour la teinture d'oxydation et parmi lesquelles on peut notamment citer les paraphénylènediamines, les bis-phénylalkylènediamines, les para-aminophénols, les ortho-aminophénols et les bases hétérocycliques. Lorsqu'elles sont utilisées, la ou les bases d'oxydation représentent de préférence de 0,0005 à 12 % en poids environ du poids total de la composition tinctoriale, et encore plus préférentiellement de 0,005 à 6 % en poids environ de ce poids.

Lorsqu'elle est destinée à la teinture d'oxydation, la composition tinctoriale conforme à l'invention peut également renfermer, en plus du colorant direct cationique (i) et de la silicone (ii) ainsi que des bases d'oxydation, un ou plusieurs coupleurs de façon à modifier ou à enrichir en reflets les nuances obtenues en mettant en oeuvre le ou les colorants direct(s) cationique(s) (i) et la ou les bases d'oxydation.

Les coupleurs utilisables dans la composition tinctoriale conforme à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques.
Lorsqu'ils sont présents, le ou les coupleurs représentent de préférence de 0,0001 à 10 % en poids environ du poids total de la composition tinctoriale et encore plus préférentiellement de 0,005 à 5 % en poids environ de ce poids.

La composition tinctoriale conforme à l'invention peut également renfermer divers adjuvants utilisés classiquement dans les compositions pour la teinture des cheveux, tels que des agents antioxydants, des agents de pénétration, des agents séquestrants, des parfums, des tampons, des agents dispersants, des agents filmogènes, des céramides, des agents conservateurs, des agents filtrants, des agents opacifiants.

Bien entendu, l'homme de l'art veillera à choisir ce ou ces éventuels composés complémentaires de manière telle que les propriétés avantageuses attachées intrinsèquement à la composition tinctoriale conforme à l'invention ne soient pas, ou substantiellement pas, altérées par la ou les adjonctions envisagées.

La composition tinctoriale selon l'invention peut se présenter sous des formes diverses, telles que sous forme de liquides, de shampooings, de crèmes, de gels, ou sous toute autre forme appropriée pour réaliser une teinture des fibres kératiniques, et notamment des cheveux humains. Elle peut être obtenue par mélange extemporané d'une composition, éventuellement pulvérulente, contenant le ou les colorants directs cationiques avec une composition contenant la silicone.

Lorsque l'association du colorant direct cationique (i) et de la silicone (ii) selon l'invention est utilisée dans une composition destinée à la teinture d'oxydation (une ou plusieurs bases d'oxydation sont alors utilisées, éventuellement en présence d'un ou plusieurs coupleurs) ou lorsqu'elle est utilisée dans une composition destinée à la teinture directe éclaircissante. alors la composition tinctoriale conforme à l'invention renferme en outre au moins un agent oxydant, choisi par exemple parmi le peroxyde d'hydrogène, le peroxyde d'urée, les bromates de métaux alcalins, les persels tels que les perborates et persulfates, et les enzymes telles que les peroxydases, les laccases et les oxydo-réductases à deux électrons. L'utilisation du peroxyde d'hydrogène ou des enzymes est particulièrement préférée.

Un autre objet de l'invention est un procédé de teinture des fibres kératiniques et en particulier des fibres kératiniques humaines telles que les cheveux mettant en oeuvre la composition tinctoriale telle que définie précédemment.

Selon une première variante de ce procédé de teinture conforme à l'invention, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Le temps nécessaire au développement de la coloration sur les fibres kératiniques est généralement compris entre 3 et 60 minutes et encore plus précisément 5 et 40 minutes.

Selon une deuxième variante de ce procédé de teinture conforme à l'invention, on applique sur les fibres au moins une composition tinctoriale telle que définie précédemment, pendant un temps suffisant pour développer la coloration désirée, sans rinçage final.

Selon une forme de réalisation particulière de ce procédé de teinture, et lorsque la composition tinctoriale conforme à l'invention renferme au moins une base d'oxydation et au moins un agent oxydant, le procédé de teinture comporte une étape préliminaire consistant à stocker sous forme séparée, d'une part, une composition (A1) comprenant, dans un milieu approprié pour la teinture, au moins un colorant direct cationique (i) tel que défini précédemment et au moins une base d'oxydation et, d'autre part, une composition (B1) renfermant, dans un milieu approprié pour la teinture. au moins un agent oxydant, puis à procéder à leur mélange au moment de l'emploi avant d'appliquer ce mélange sur les fibres kératiniques, la composition (A1) ou la composition (B1) contenant la silicone (ii) telle que définie précédemment.

Selon une autre forme de réalisation particulière de ce procédé de teinture, et lorsque la composition tinctoriale conforme à l'invention renferme au moins un agent oxydant, le procédé de teinture comporte une étape préliminaire consistant à stocker sous forme séparée, d'une part, une composition (A2) comprenant, dans un milieu approprié pour la teinture, au moins un colorant direct cationique (i) tel que défini précédemment et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant la silicone telle que définie précédemment.

Un autre objet de l'invention est un dispositif à plusieurs compartiments ou "kit" de teinture ou tout autre système de conditionnement à plusieurs compartiments dont un premier compartiment renferme la composition (A1) ou (A2) telle que définie ci-dessus et un second compartiment renferme la composition (B1) ou (B2) telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLE 1 :

On a préparé la composition de teinture directe du tableau suivant :
(*toutes teneurs exprimées en grammes*)

| **EXEMPLES N°→** | **1** |
|---|---|
| Colorant direct cationique de formule (I1). | 0,10 |
| Colorant direct cationique de formule(114) | 0,10 |
| Nonyl phénol à 9 moles d'oxyde d'éthylène.... | 8,0 |
| Silicone aminée (mélange de polydiméthylsiloxane à groupements aminoéthyl-aminoisobutyle/polydiméthylsiloxane), vendue sous la dénomination Q2-8220 par la société OSI. | 1,2 |
| Silicone polyoxyalkylénée (polydiméthylsiloxane oxyéthylénée à 22 OE et oxypropylénée à 23 OP), vendue sous la dénomination MIRASIL DMCO par la société RHODIA CHIMIE ........... | |
| Ethanol..... | 10 |
| 2-amino-2-méthyl-1-propanol .....qs..... | pH 9 |
| Eau déminéralisée .qsp..... | 100 |

La composition ci-dessus a été appliquée pendant 30 minutes sur des mèches de cheveux gris naturels à 90% de blancs. Les mèches de cheveux ont ensuite été rincées, lavées avec un shampooing standard puis séchées.

Les mèches ont été teintes dans les nuances suivantes :

| **Exemple** | **Nuance obtenue** |
|---|---|
| 1 | Rouge orangé puissant |

## Revendications

1. Composition pour la teinture des fibres kératiniques et en particulier des fibres kératiniques humaines telles que les cheveux, renfermant dans un milieu approprié pour la teinture, (i)au moins composé choisi parmi ceux de formules (I) ou (III), suivantes :
**a) les composés de formule (I) suivante :** 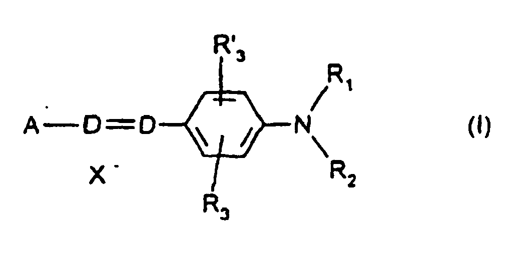 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄ , alcoxy en C₁-C₄ ou acétyloxy,
X ⁻ représente un anion de préférence choisi parmi le chlorure, le méthyl sulfate et l'acétate,
A représente un groupement choisi par les structures A1 à A18 suivantes : 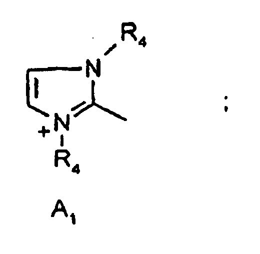 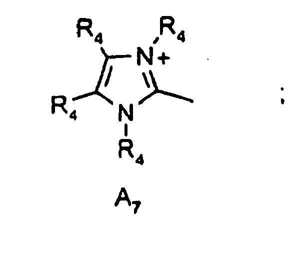 et 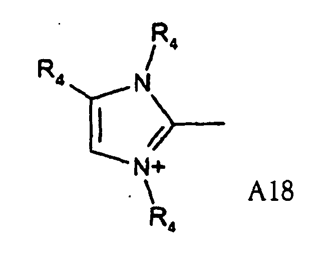
dans lesquelles R₄ représente un radical alkyle en C₁-C₄ pouvant être substitué par un radical hydroxyle et R₅ représente un radical alcoxy en C₁-C₄, sous réserve que lorsque D représente -CH, que A représente A₄ ou A₁₃ et que R₃ est différent d'un radical alcoxy, alors R₁ et R₂ ne désignent pas simultanément un atome d'hydrogène ;
**c) les composés de formule (III) suivante :** 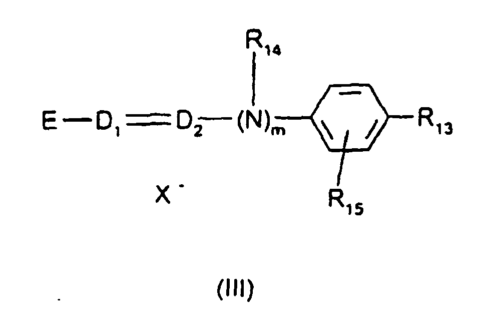 dans lesquelles :
R₁₃ représente un atome d'hydrogène, un radical alcoxy en C₁-C₄, un atome d'halogène tel que le brome, le chlore, l'iode ou le fluor ou un radical amino,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
D₁ et D₂, identiques ou différents, représentent un atome d'azote ou le groupement -CH,
m = 0 ou 1,
étant entendu que lorsque R₁₃ représente un groupement amino non substitué, alors D₁ et D₂ représentent simultanément un groupement -CH et m = 0,
X ⁻ représente un anion de préférence choisi parmi le chlorure, le méthyl sulfate et l'acétate,
E représente un groupement choisi par les structures E1 à E8 suivantes : 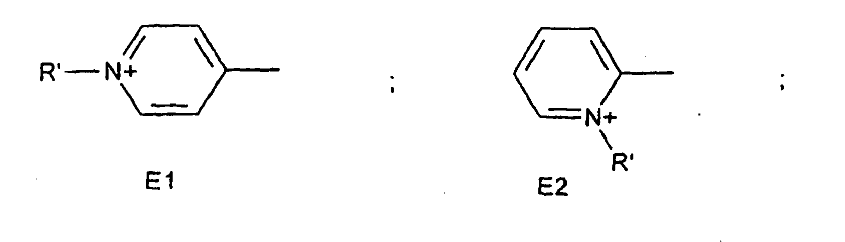 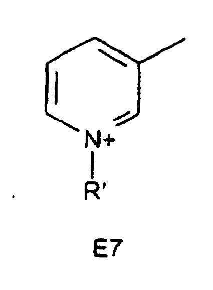 dans lesquelles R' représente un radical alkyle en C₁-C₄ ;
ladite composition étant **caractérisée par le fait qu'**elle contient en outre une silicone choisie dans le groupe constitué par:
**(ii)₁** les silicones aminées,
**(ii)₂** les silicones polyoxyalkylénées,
**(ii)₃** les gommes et résines de silicones.

2. Composition selon la revendication 1, **caractérisée par le fait que** les colorants directs cationiques de formule (I) sont choisis parmi les composés répondant aux structures (I1) à (I54) suivantes :

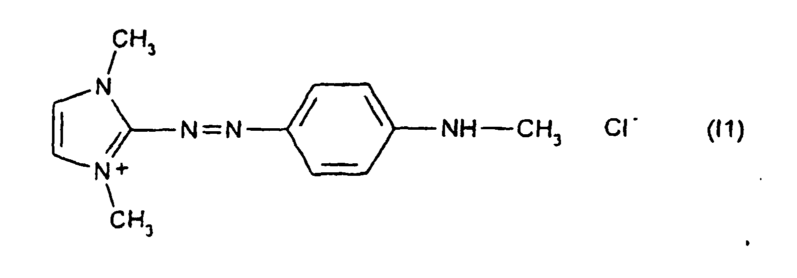

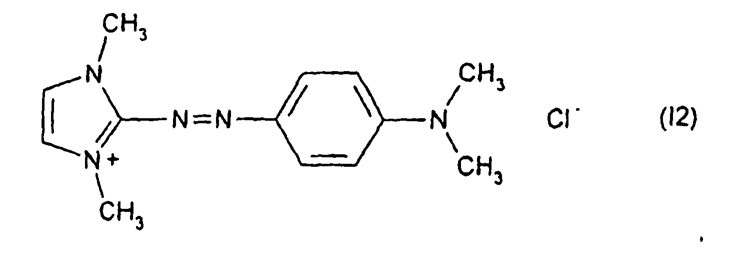

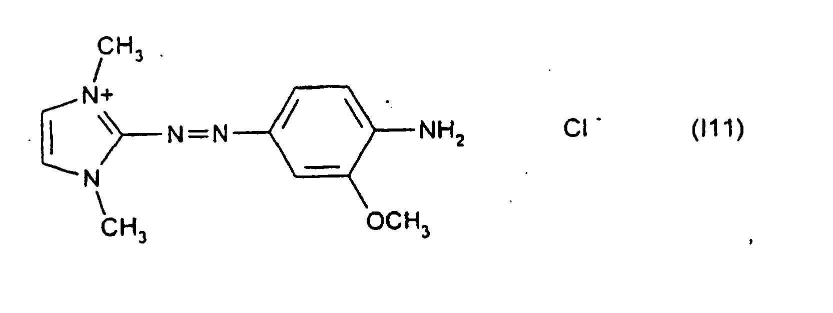

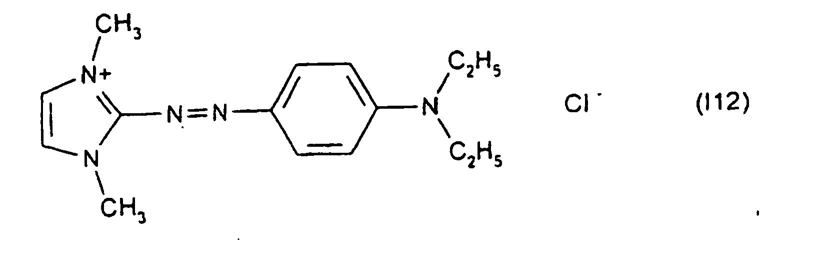

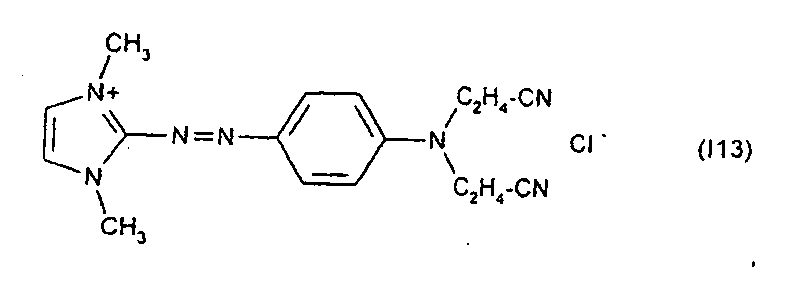

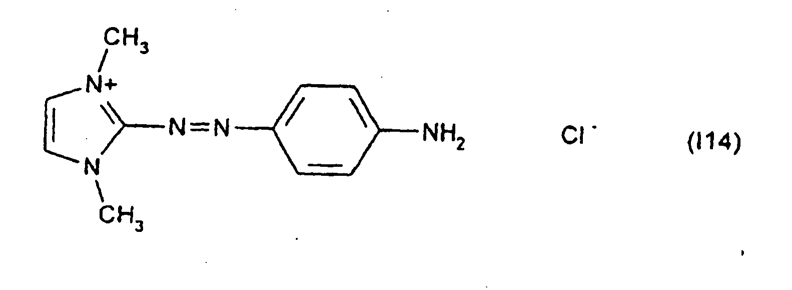

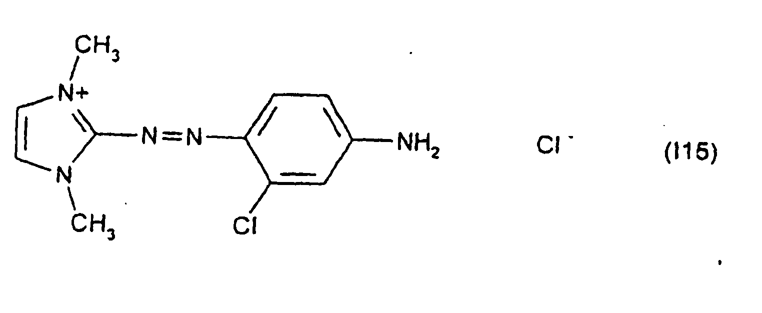

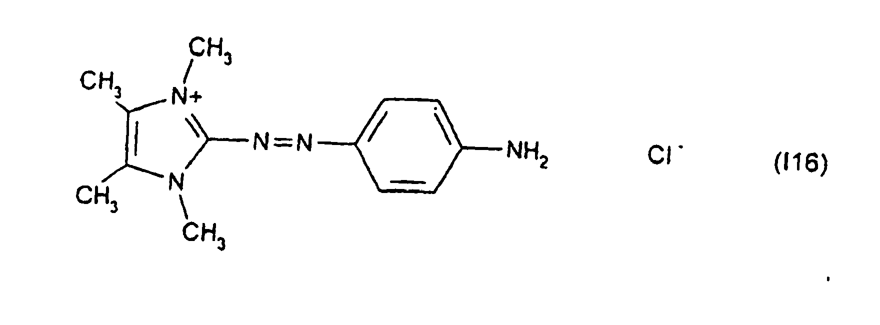

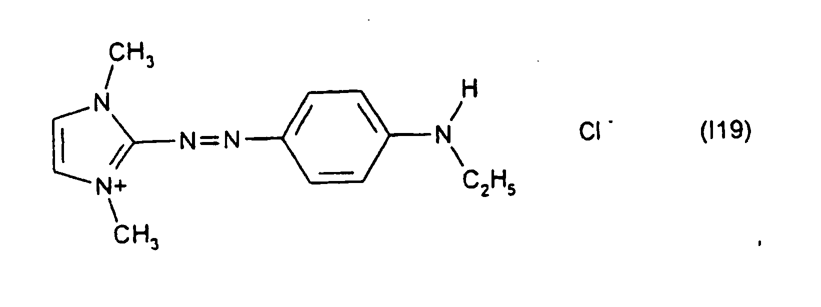

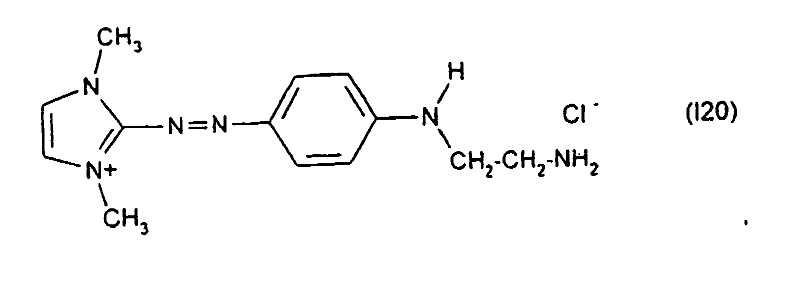

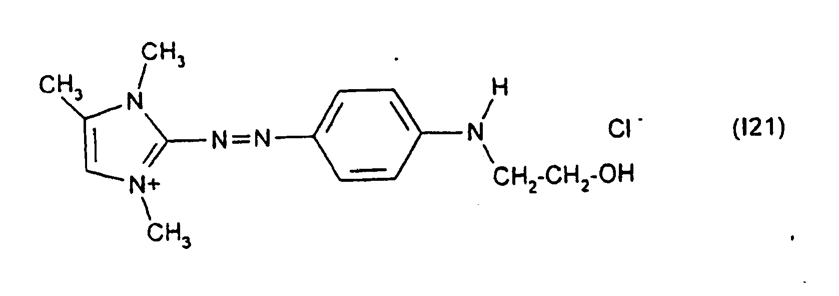

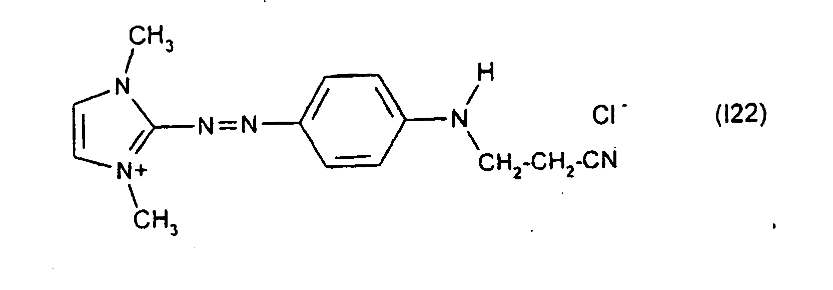

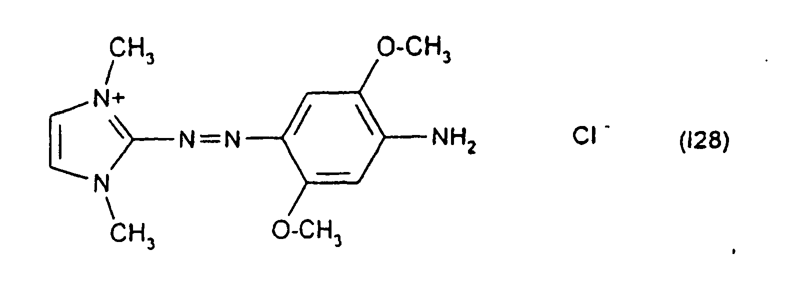

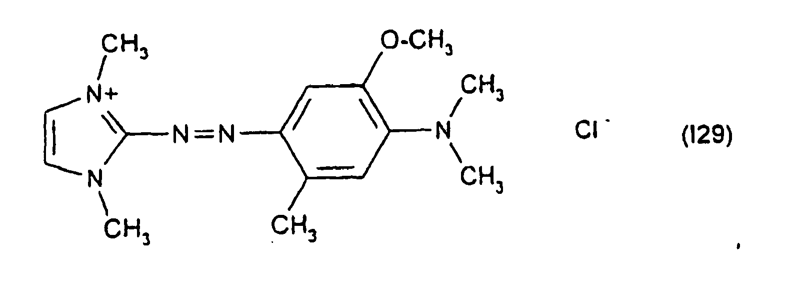

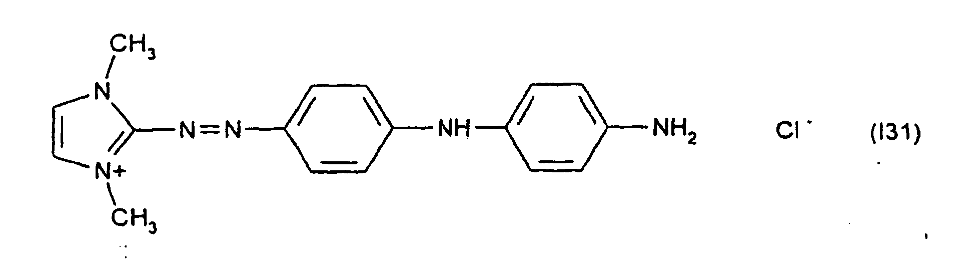

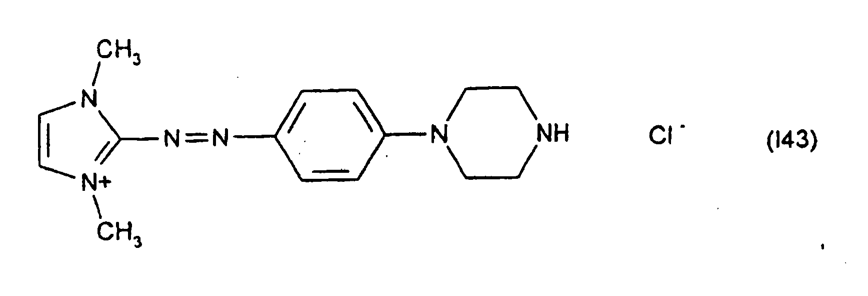

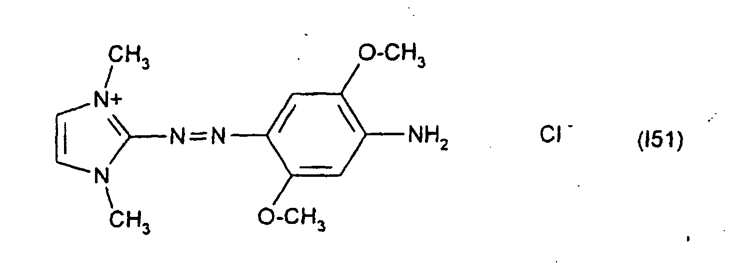

3. Composition selon la revendication 2, **caractérisée par le fait que** les colorants directs cationiques répondent aux structures (I1), (I2), (I14), et (I31).

4. Composition selon la revendication 1, **caractérisée par le fait que** les colorants directs cationiques de formule (III) sont choisis parmi les composés répondant aux structures (III1) à (III18) suivantes :

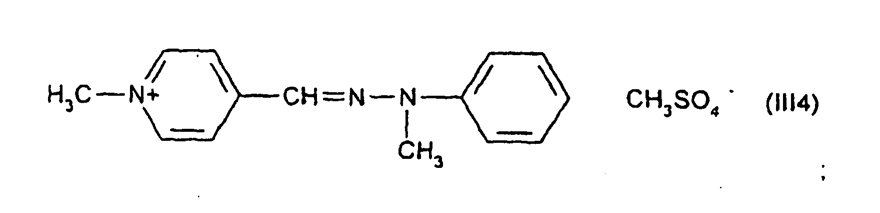

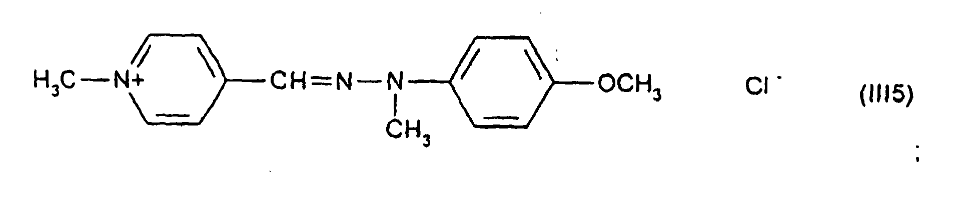

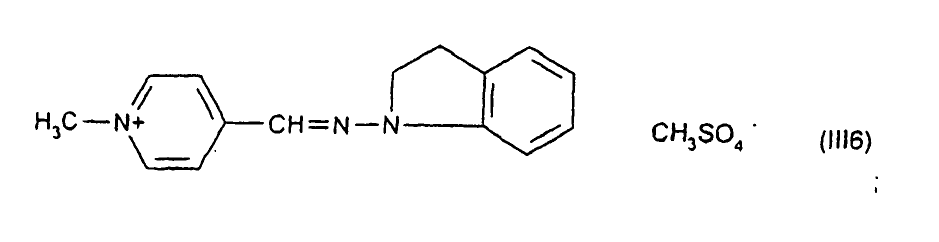

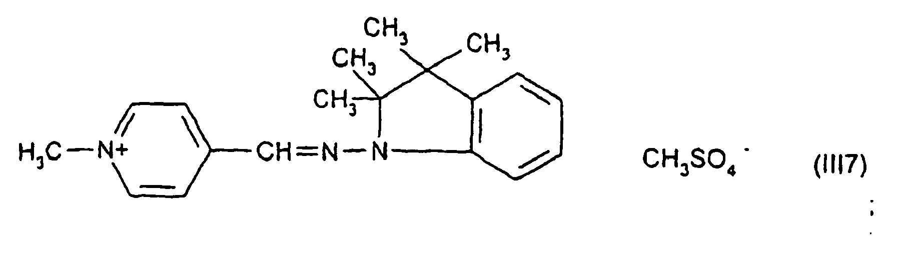

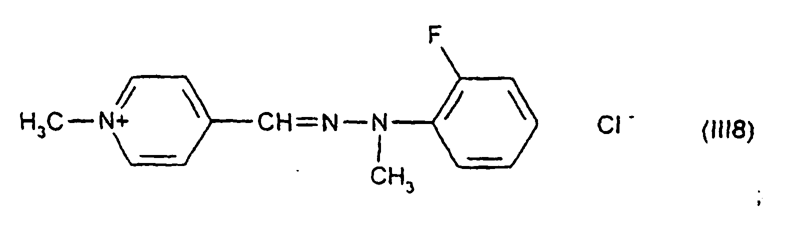

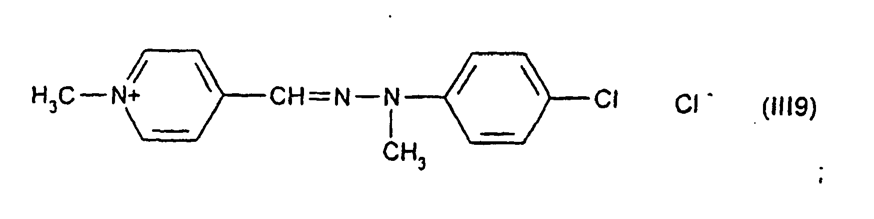

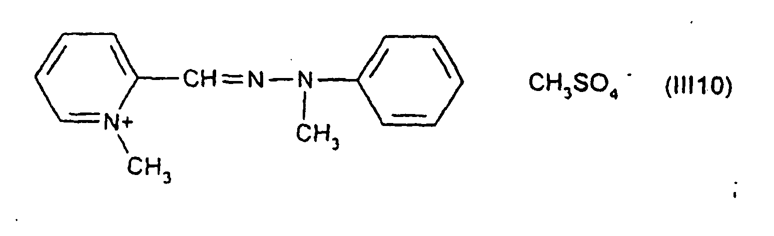

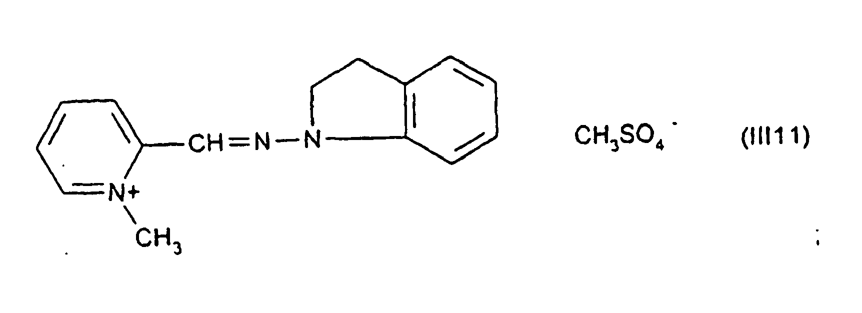

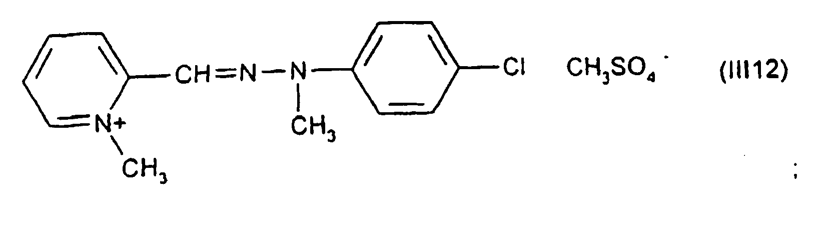

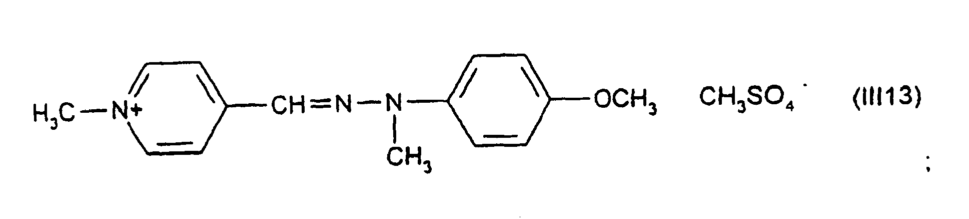

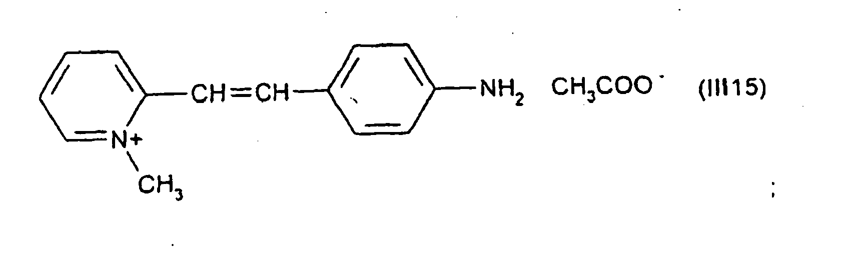

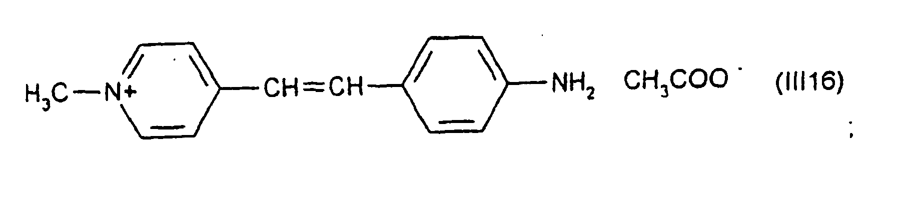

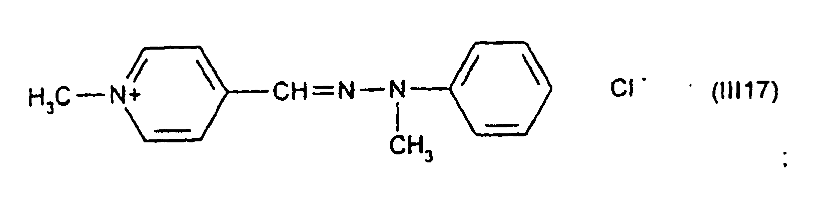

5. Composition selon la revendication 4, **caractérisée par le fait que** les colorants directs cationiques de formule (III) sont choisis parmi les composés répondant aux structures (III4), (III5) et (III13).

6. Composition selon l'une quelconque des revendications précédentes, **caractérisée par le fait que** le ou les colorants directs cationiques de formules (I), ou (III), représentent de 0,001 à 10 % en poids du poids total de la composition.

7. Composition selon la revendication 6, **caractérisée par le fait que** le ou les colorants directs cationiques de formules (I), ou (III) représentent de 0,005 à 5 % en poids du poids total de la composition.

8. Composition selon l'une quelconque des revendications précédentes, **caractérisée par le fait que** la silicone aminée (ii)₁ est choisie parmi les composés de formule (V) suivante: 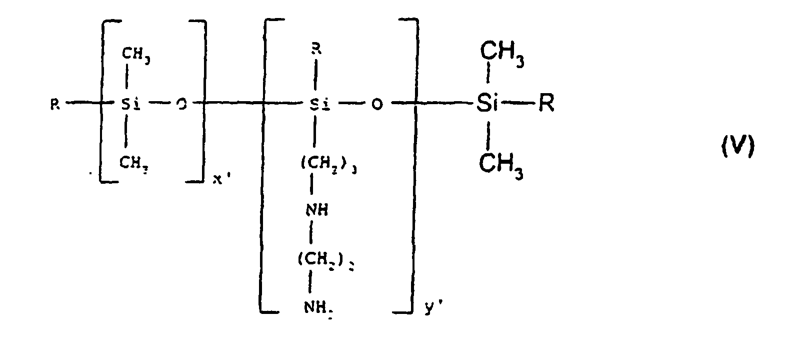 dans laquelle R désigne le radical CH₃ ou OH, et
x' et y' sont des nombres entiers dépendant du poids moléculaire.

9. Composition selon l'une quelconque des revendications 1 à 7, **caractérisée par** le fait la silicone aminée (ii)₁ est choisie parmi les composés de formule (VI) suivante:
(R¹)ₐ (T)₃₋ₐ -Si[OSi(T)₂]ₙ -[OSi(T)_{b}(R¹)_{2-b}]ₘ -OSi(T)₃₋ₐ - (R¹)ₐ **(VI)**
dans laquelle,
T est un atome d'hydrogène, ou un radical phényle. ou OH, ou alkyle en C₁-C₈, et de préférence méthyle,
a désigne le nombre 0 ou un nombre entier de 1 à 3, et de préférence 0,
b désigne 0 ou 1, et en particulier 1,
m et n sont des nombres tels que la somme (n + m) varie notamment de 1 à 2 000 et en particulier de 50 à 150, n désignant un nombre de 0 à 1 999 et notamment de 49 à 149 et m désignant un nombre de 1 à 2 000, et notamment de 1 à 10 ;
R¹ est un radical monovalent de formule -C_{q}H_{2Q}L dans laquelle q est un nombre de 2 à 8 et L est un groupement aminé éventuellement quaternisé choisi parmi les groupements : -N(R²)-CH₂-CH₂-N(R²)₂ ; -N(R²)₂ ; -N^{⊕}(R²)₃Q⁻ ; -N^{⊕}(R²)(H)₂Q⁻ ; -N^{⊕}(R²)₂HQ⁻ ; -N(R²)-CH₂-CH₂-N^{⊕}(R²)(H)₂ Q⁻,
dans lesquels, R² désigne hydrogène, phényle, benzyle, ou un radical hydrocarboné saturé monovalent, de préférence un radical alkyle ayant de 1 à 20 atomes de carbone, et Q⁻ représente un ion halogénure.

10. Composition selon la revendication 9, **caractérisée par le fait que** la silicone aminée est choisie parmi les composés de formule (VII) suivante: 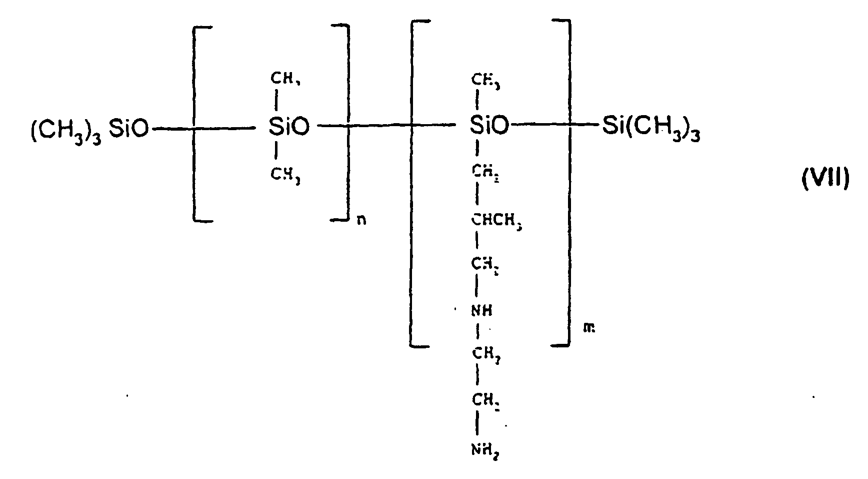 dans laquelle n et m ont les significations données pour la formule (VI).

11. Composition selon l'une quelconque des revendications 1 à 7, **caractérisée par le fait que** la silicone aminée (ii)₁ est choisie parmi les composés de formule (VIII) suivante: 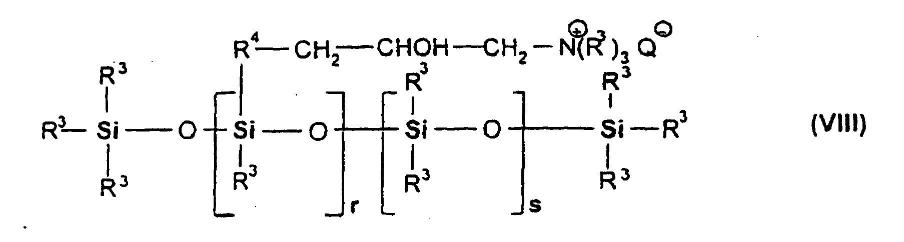 dans laquelle,
R³ représente un radical hydrocarboné monovalent ayant de 1 à 18 atomes de carbone, et de préférence un radical alkyle en C₁-C₁₈, ou alcényle en C₂-C₁₈;
R⁴ représente un radical hydrocarboné divalent, de préférence un radical alkylène en C₁-C₁₈ ou un radical alkylèneoxy divalent en C₁-C₁₈;
Q⁻ est un ion halogénure;
r représente une valeur statistique moyenne de 2 à 20 et de préférence de 2 à 8;
s représente une valeur statistique moyenne de 20 à 200 et de préférence de 20 à 50.

12. Composition selon l'une quelconque des revendications 1 à 7, **caractérisée par le fait que** la silicone polyoxyalkylénée (ii)₂ est choisie parmi les composés de formule (IX), (X), (XI), et (XII) suivantes: 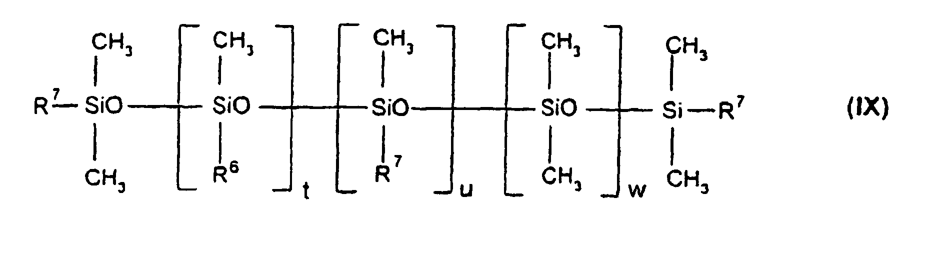 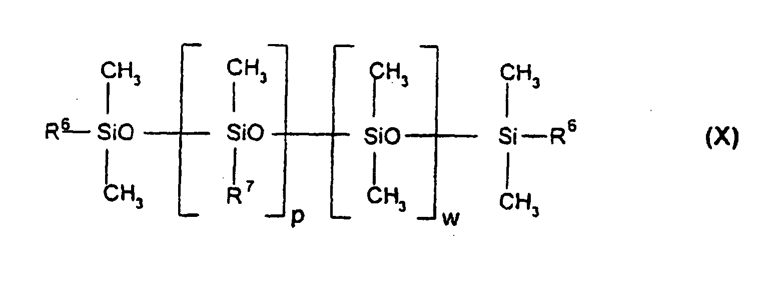 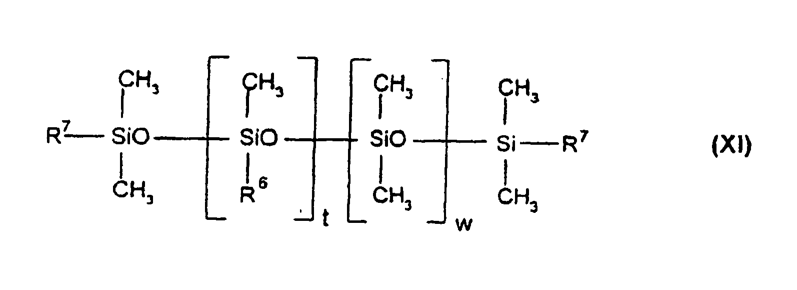 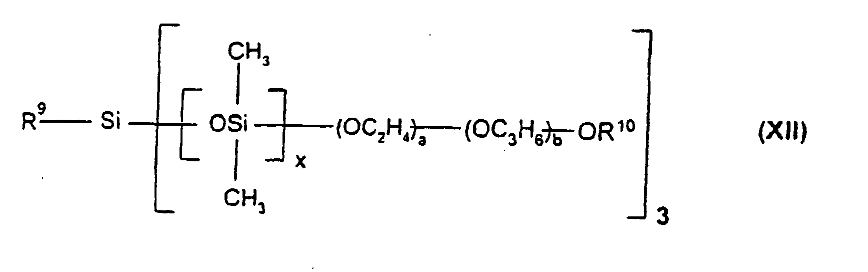 formules (IX), (X), (XI) et (XII) dans lesquelles,
- R⁶, identique ou différent, représente un radical alkyle, linéaire ou ramifié, en C₁-C₃₀ ou phényle,
- R⁷, identique ou différent, représente un radical -C_{C}H_{2c}-O-(C₂H₄O)ₐ(C₃H₆O)_{b}-R⁸ ou un radical -C_{c}H_{2c}-O-(C₄H₈0)ₐ-R⁸,
- R⁹, R¹⁰ identiques ou différents, désignent un radical alkyle, linéaire ou ramifié, en C₂-C₁₂, et de préférence méthyle,
- R⁸, identique ou différent, est choisi parmi un atome d'hydrogène, un radical alkyle, linéaire ou ramifié, en C₁-C₁₂, un radical alcoxy, linéaire ou ramifié, en C₁-C₆, un radical acyle, linéaire ou ramifié, en C₂-C₃₀, un radical hydroxyle, -SO₃M, aminoalcoxy en C₁-C₆ éventuellement substitué sur l'amine, aminoacyle en C₂-C₆ éventuellement substitué sur l'amine, -NHCH₂CH₂COOM, -N(CH₂CH₂COOM)₂, aminoalkyle éventuellement substitué sur l'amine et sur la chaîne alkyle, carboxyacyle en C₂-C₃₀, un groupement phosphono éventuellement substitué par un ou deux radicaux aminoalkyle substitués, -CO(CH₂)_{d}COOM, -COCHR¹¹ (CH₂)_{d}COOM, -NHCO(CH₂)_{d}OH, -NH₃Y, un groupement phosphate,
- M, identique ou différent, désigne un atome d'hydrogène, Na, K, Li, NH₄ ou une amine organique,
- R¹¹ désigne un atome d'hydrogène ou un radical -SO₃M,
- d varie de 1 à 10,
- u varie de 0 à 20,
- w varie de 0 à 500,
- t varie de 0 à 20,
- p varie de 1 à 50,
- a varie de 0 à 50,
- b varie de 0 à 50,
- la somme (a + b) est supérieure ou égale à 2,
- c varie de 0 à 4,
- x varie de 1 à 100,
- Y représente un anion minéral ou organique monovalent.

13. Composition selon la revendication 12, **caractérisée par le fait que** la silicone est choisie parmi celles de formules (X) ou (XI) dans lesquelles:
- c est égal à 2 ou 3;
- R⁶ désigne méthyle;
- R⁸ représente un radical méthyle, un radical acyle en C₁₂-C₂₂, CO(CH₂)_{d}COOM;
- a varie de 2 à 25 et de préférence de 2 à 15.
- b est égal à 0.
- w varie de 0 à 100.
- p varie de 1 à 20.

14. Composition selon l'une quelconque des revendications 1 à 7, **caractérisée par le fait que** la silicone polyoxyalkylénée (ii)₂ est choisie parmi les composés de formule (XIII) suivante:
([Z(R¹²SiO)_{q}R¹³SiZO] [(CₙH₂ₙO)ᵣ])ₛ **(XIII)**
dans laquelle,
- R¹² et R¹³, identiques ou différents, représentent un radical hydrocarboné monovalent, n est un nombre entier allant de 2 à 4, q est un nombre supérieur ou égal à 4, de préférence compris entre 4 et 200 , r est un nombre supérieur ou égal à 4, de préférence compris entre 4 et 200, s est un nombre supérieur ou égal à 4, de préférence compris entre 4 et 1 000, Z représente un groupe organique divalent qui est lié à l'atome de silicium adjacent par une liaison carbone-silicium et à un bloc polyoxyalkylène par un atome d'oxygène,
le poids moléculaire moyen de chaque bloc siloxane étant compris entre 400 et 10.000, celui de chaque bloc polyoxyalkylène étant compris entre 300 et 10.000, les blocs siloxane représentant de 10 à 95% en poids du copolymère bloc, et le poids moléculaire moyen en nombre du copolymère bloc allant de 2.500 à 1.000.000, et de préférence de 3.000 à 200.000.

15. Composition selon l'une quelconque des revendications précédentes, **caractérisée par le fait que** la ou les silicones (ii) représentent de 0,01 à 20% en poids environ du poids total de la composition tinctoriale.

16. Composition selon la revendication 15, **caractérisée par le fait que** la ou les silicones représentent de 0,1 à 10% en poids environ du poids total de la composition tinctoriale.

17. Composition selon l'une quelconque des revendications précédentes, **caractérisée par le fait que** le milieu approprié pour la teinture (ou support) est constitué par de l'eau ou par un mélange d'eau et d'au moins un solvant organique.

18. Composition selon l'une quelconque des revendications précédentes, **caractérisée par le fait qu'**elle présente un pH compris entre 2 et 11, et de préférence entre 5 et 10.

19. Composition selon l'une quelconque des revendications précédentes, **caractérisée par le fait qu'**elle est destinée à la teinture d'oxydation et qu'elle contient une ou plusieurs bases d'oxydation choisie parmi les paraphénylènediamines, les bis-phénylalkylènediamines, les para-aminophénols, les ortho-aminophénols et les bases hétérocycliques.

20. Composition selon la revendication 19, **caractérisée par le fait que** la ou les bases d'oxydation représentent 0,0005 à 12 % en poids du poids total de la composition tinctoriale.

21. Composition selon la revendication 20, **caractérisée par le fait que** la ou les bases d'oxydation représentent 0,005 à 6 % en poids du poids total de la composition tinctoriale.

22. Composition selon l'une quelconque des revendications 19 à 21, **caractérisée par le fait qu'**elle renferme un ou plusieurs coupleurs choisis parmi les métaphénylènediamines, les méta-aminophénols, les métadiphénols et les coupleurs hétérocycliques.

23. Composition selon la revendication 22, **caractérisée par le fait que** le ou les coupleurs représentent de 0,0001 à 10 % en poids du poids total de la composition tinctoriale.

24. Composition selon la revendication 23, **caractérisée par le fait que** le ou les coupleurs représentent de 0,005 à 5 % en poids du poids total de la composition tinctoriale.

25. Composition selon l'une quelconque des revendications précédentes, **caractérisée par le fait qu'**elle est destinée à la teinture directe éclaircissante ou la teinture d'oxydation et qu'elle renferme alors au moins un agent oxydant.

26. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 25, pendant un temps suffisant pour développer la coloration désirée, après quoi on rince, on lave éventuellement au shampooing, on rince à nouveau et on sèche.

27. Procédé de teinture des fibres kératiniques et en particulier des fibres kératiniques humaines telles que les cheveux, **caractérisé par le fait que** qu'on applique sur les fibres au moins une composition tinctoriale telle que définie à l'une quelconque des revendications 1 à 25, pendant un temps suffisant pour développer la coloration désirée, sans rinçage final.

28. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A1) comprenant, dans un milieu approprié pour la teinture, au moins un colorant direct cationique (i) tel que défini dans les revendications précédentes et au moins une base d'oxydation et, d'autre part, une composition (B1) renfermant, dans un milieu approprié pour la teinture, au moins un agent oxydant, puis à procéder à leur mélange au moment de l'emploi avant d'appliquer ce mélange sur les fibres kératiniques, la composition (A1) ou la composition (B1) contenant la silicone (ii) telle que définie dans les revendications précédentes.

29. Procédé de teinture des fibres kératiniques et en particulier des fibres kératiniques humaines telles que les cheveux, **caractérisé par le fait qu'**il comporte une étape préliminaire consistant à stocker sous forme séparée, d'une part, une composition (A2) comprenant, dans un milieu approprié pour la teinture, au moins un colorant direct cationique (i) tel que défini dans les revendications précédentes et, d'autre part, une composition (B2) renfermant, dans un milieu approprié pour la teinture, au moins un agent oxydant, puis à procéder à leur mélange au moment de l'emploi avant d'appliquer ce mélange sur les fibres kératiniques, la composition (A2) ou la composition (B2) contenant la silicone (ii) telle que définie dans les revendications précédentes.

30. Dispositif à plusieurs compartiments ou "kit" de teinture à plusieurs compartiments, **caractérisé par le fait qu'**un premier compartiment renferme la composition (A1) ou (A2) telle que définie à la revendication 28 ou 29 et un second compartiment renferme la composition (B1) ou (B2) telle que définie à la revendication 28 ou 29.

## Claims

1. Composition for dyeing keratinous fibres and in particular human keratinous fibres such as hair, containing, in an appropriate dyeing medium, (i) at least one compound chosen from those of the following formulae (I) or (III):
a) the compounds of the following formula (I) : 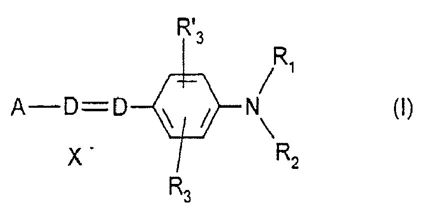 in which:
D represents a nitrogen atom or the -CH group,
R₁ and R₂, which are identical or different, represent a hydrogen atom; a C₁-C₄ alkyl radical which may be substituted with a -CN, -OH or -NH₂ radical or form with a carbon atom of the benzene ring an optionally oxygen-containing or nitrogen-containing heterocycle which may be substituted with one or more C₁-C₄ alkyl radicals; a 4'-aminophenyl radical,
R₃ and R'₃, which are identical or different, represent a hydrogen or halogen atom chosen from chlorine, bromine, iodine and fluorine, a cyano, C₁-C₄ alkyl, C₁-C₄ alkoxy or acetyloxy radical,
X⁻ represents an anion which is preferably chosen from chloride, methylsulphate and acetate,
A represents a group chosen from the following structures A₁, A₇ and A₁₈: 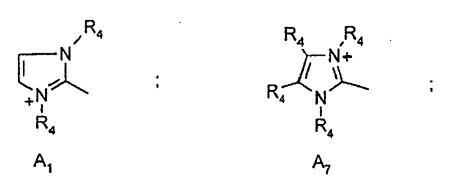 and 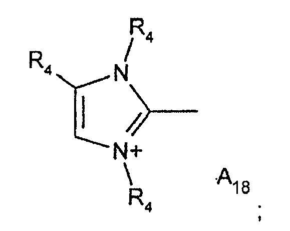 in which R₄ represents a C₁-C₄ alkyl radical which may be substituted with a hydroxyl radical and R₅ represents a C₁-C₄ alkoxy radical, with the proviso that when D represents -CH, A represents A₄ or A₁₃ and R₃ is different from an alkoxy radical, then R₁ and R₂ do not simultaneously denote a hydrogen atom;
b) the compounds of the following formula (III): 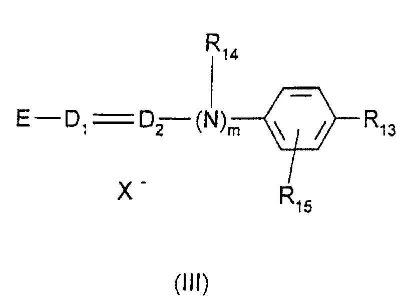 in which:
R₁₃ represents a hydrogen atom, a C₁-C₄ alkoxy radical, a halogen atom such as bromine, chlorine, iodine or fluorine or an amino radical,
R₁₄ represents a hydrogen atom, a C₁-C₄ alkyl radical or forms with a carbon atom of the benzene ring a heterocycle which is optionally oxygen-containing and/or substituted with one or more C₁-C₄ alkyl groups,
R₁₅ represents a hydrogen or halogen atom such as bromine, chlorine, iodine or fluorine,
D₁ and D₂, which are identical or different, represent a nitrogen atom or the -CH group,
m = 0 or 1,
it being understood that when R₁₃ represents an unsubstituted amino group, then D₁ and D₂ simultaneously represent a -CH group and m = 0,
X⁻ represents an anion which is preferably chosen from chloride, methylsulphate and acetate,
E represents a group chosen from the following structures E1, E2 and E7: 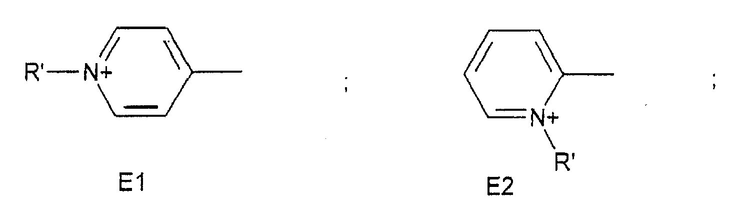 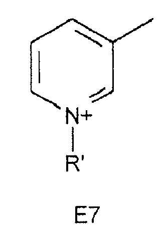 in which R' represents a C₁-C₄ alkyl radical;
the said composition being **characterized in that** it contains, in addition, a silicone chosen from the group consisting of:
(ii)₁ aminated silicones,
(ii)₂ polyoxyalkylenated silicones,
(ii)₃ silicone gums and resins.

2. Composition according to Claim 1, **characterized in that** the cationic direct dyes of formula (I) are chosen from the compounds corresponding to the following structures (I1) to (I54) :

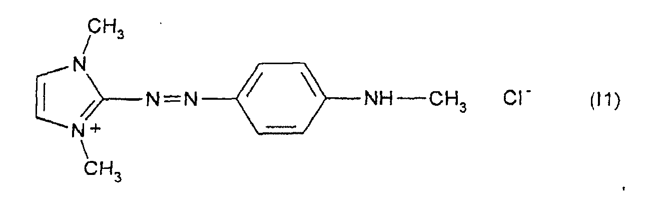

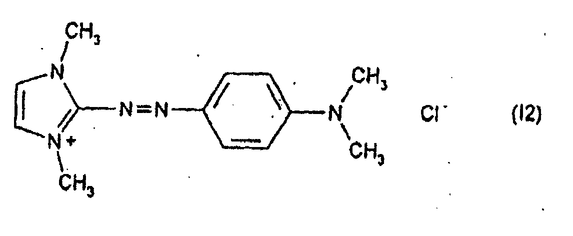

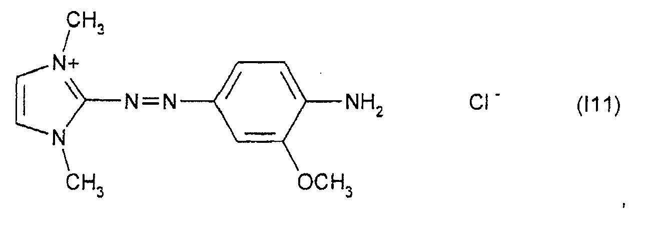

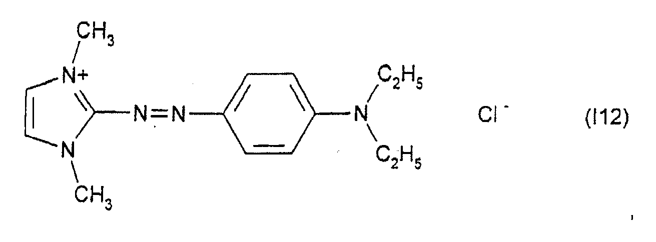

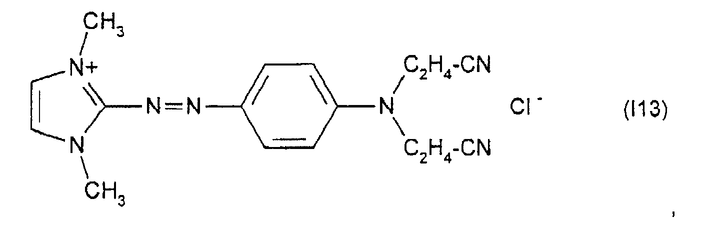

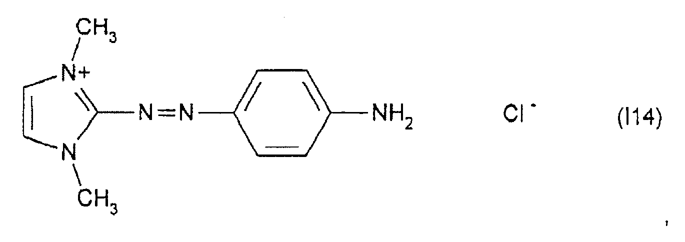

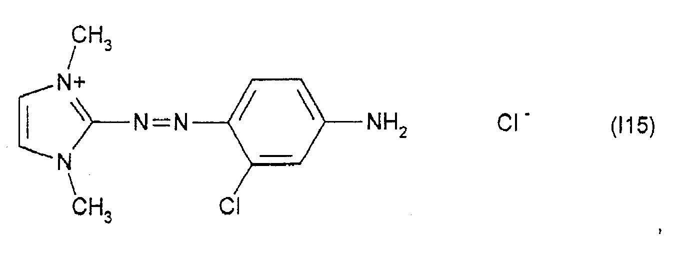

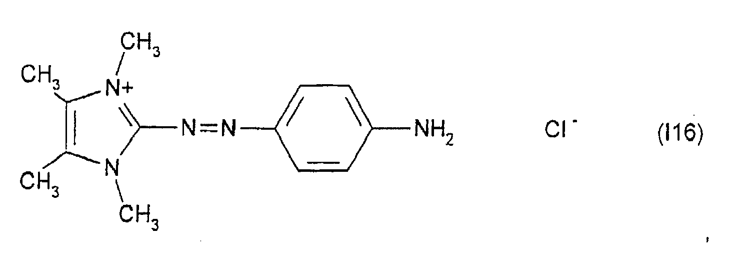

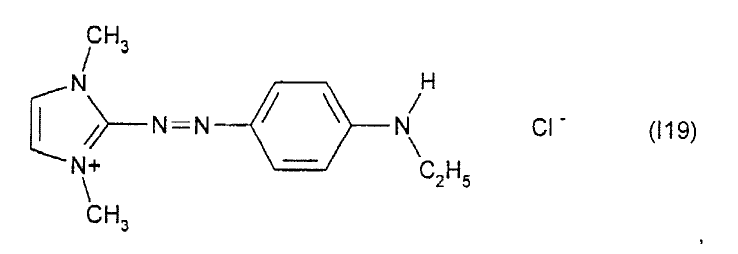

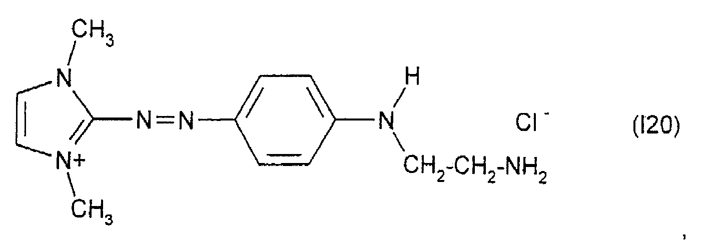

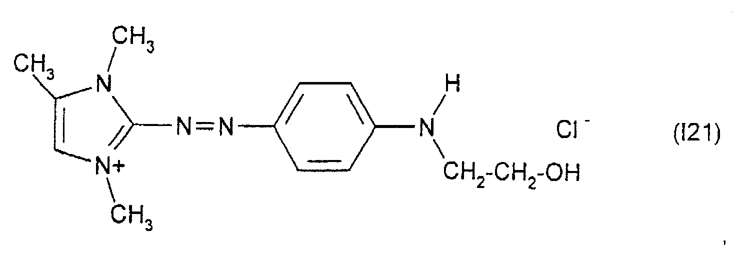

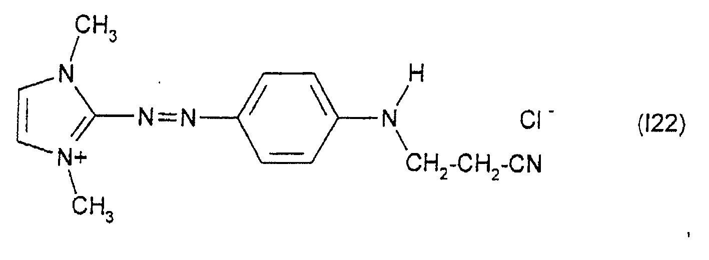

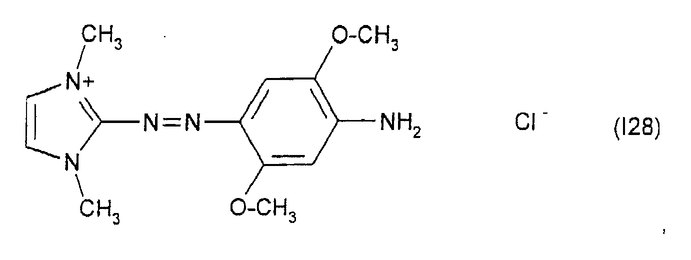

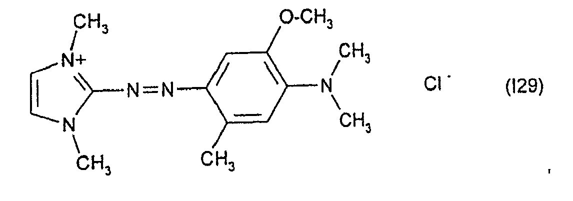

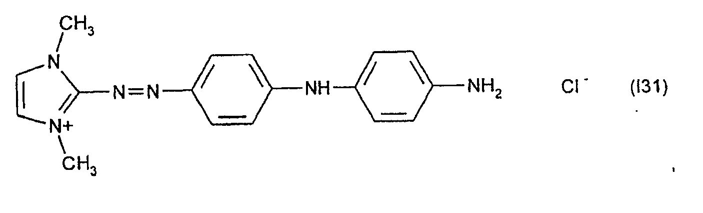

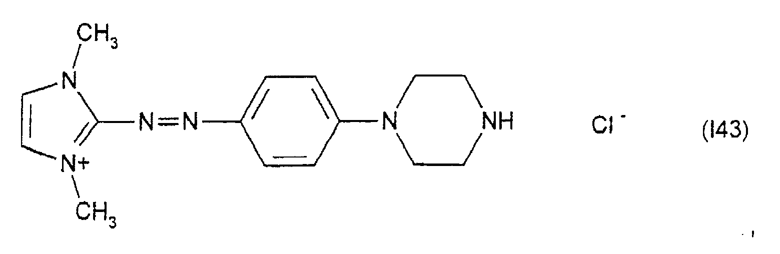

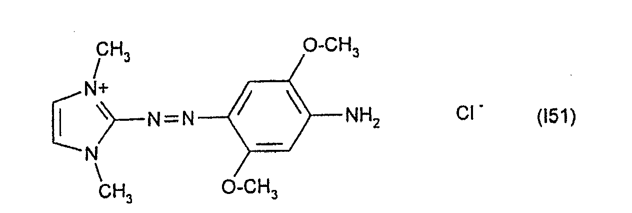

3. Composition according to Claim 2, **characterized in that** the cationic direct dyes correspond to the structures (I1), (I2), (I14), and (I31).

4. Composition according to Claim 1, **characterized in that** the cationic direct dyes of formula (III) are chosen from the compounds corresponding to the following structures (III1) to (III18) : 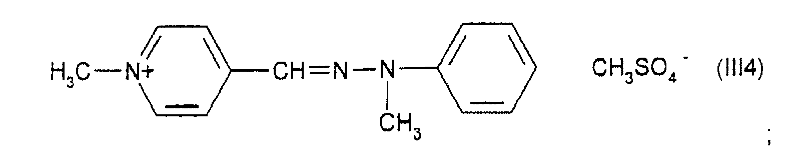 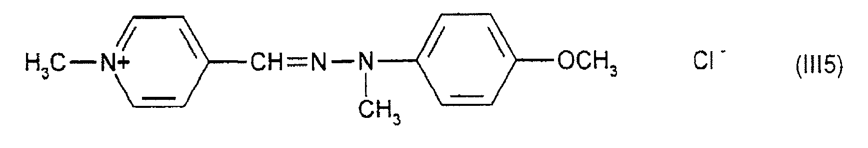 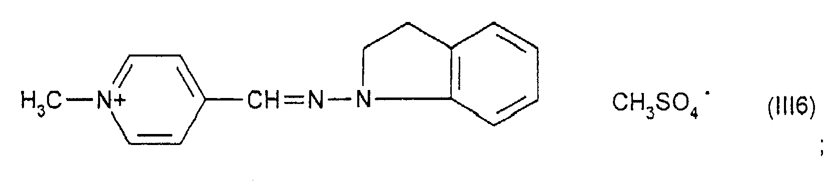 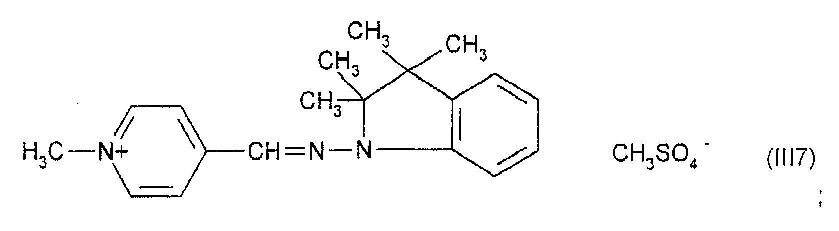 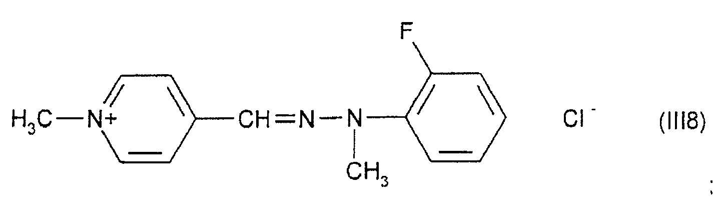 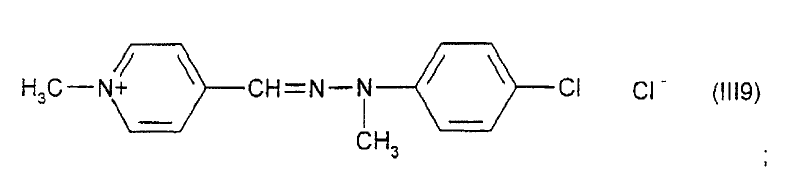 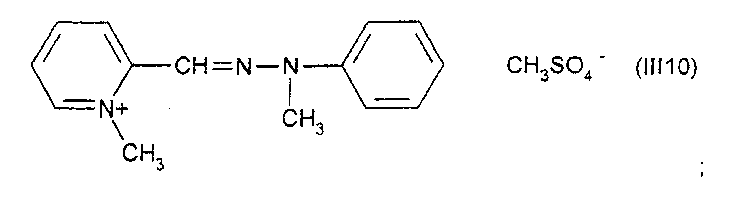 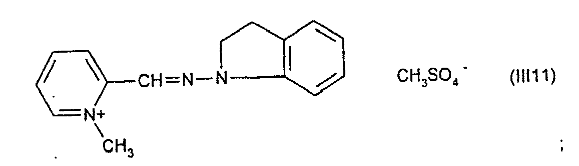 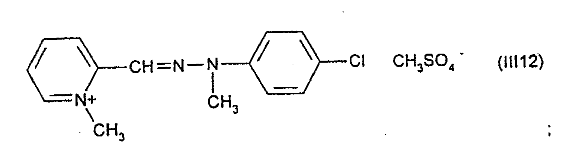 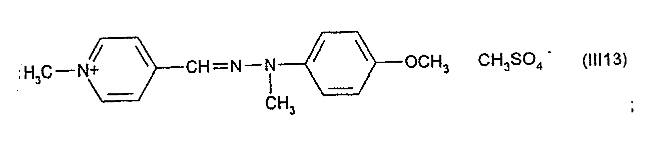 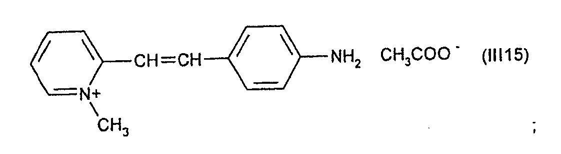 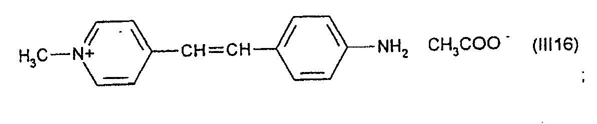 and

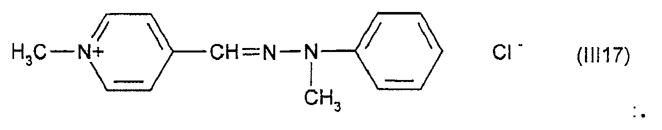

5. Composition according to Claim 4, **characterized in that** the cationic direct dyes of formula (III) are chosen from the compounds corresponding to the structures (III4), (III5) and (III13).

6. Composition according to any one of the preceding claims, **characterized in that** the cationic direct dye(s) of formulae (I) or (III) represent from 0.001 to 10% by weight of the total weight of the composition.

7. Composition according to Claim 6, **characterized in that** the cationic direct dye(s) of formulae (I) or (III) represent from 0.005 to 5% by weight of the total weight of the composition.

8. Composition according to any one of the preceding claims, **characterized in that** the aminated silicone (ii)₁ is chosen from the compounds of the following formula (V): 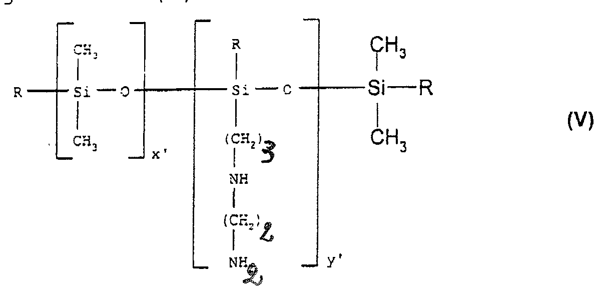
in which R denotes the CH₃ or OH radical, and
x' and y' are integers which depend on the molecular weight.

9. Composition according to any one of Claims 1 to 7, **characterized in that** the aminated silicone (ii)₁ is chosen from the compounds of the following formula (VI):
(R¹)ₐ(T)₃₋ₐ-Si[OSi(T)₂]ₙ-[OSi(T)_{b}(R¹)_{2-b}]ₘ-OSi(T)₃₋ₐ-(R¹)ₐ **(VI)**
in which,
T is a hydrogen atom, or a phenyl, or OH, or C₁-C₈ alkyl, and preferably methyl, radical,
a denotes the number 0 or an integer from 1 to 3, and preferably 0,
b denotes 0 or 1, and in particular 1,
m and n are numbers such that the sum (n + m) varies in particular from 1 to 2000 and in particular from 50 to 150, n denoting a number from 0 to 1999 and in particular from 49 to 149 and m denoting a number from 1 to 2000, and in particular from 1 to 10;
R¹ is a monovalent radical of formula -C_{q}H_{2q}L in which q is a number from 2 to 8 and L is an optionally quaternized amino group chosen from the groups:
-N(R²)-CH₂-CH₂-N(R²)₂; -N(R²)₂; -N^{⊕}(R²)₃Q⁻; -N^{⊕}(R²) (H)₂Q⁻; -N^{⊕}(R₂)₂HQ⁻ ; -N(R²)-CH₂CH₂N^{⊕}(R²) (H)₂Q⁻,
in which R² denotes hydrogen, phenyl, benzyl, or a monovalent saturated hydrocarbon radical, preferably an alkyl radical having from 1 to 20 carbon atoms and Q⁻ represents a halide ion.

10. Composition according to Claim 9, **characterized in that** the aminated silicone is chosen from the compounds of the following formula (VII): 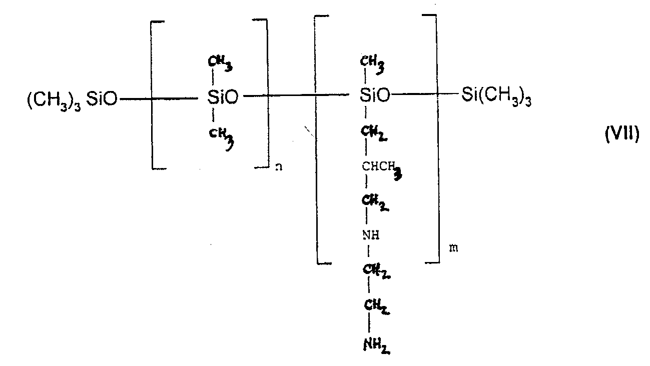 in which n and m have the meanings given for the formula (VI).

11. Composition according to any one of Claims 1 to 7, **characterized in that** the aminated silicone (ii)₁ is chosen from the compounds of the following formula (VIII): 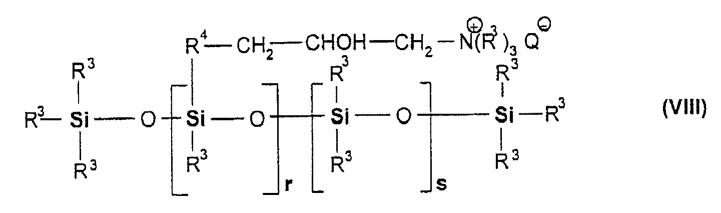 in which,
R³ represents a monovalent hydrocarbon radical having from 1 to 18 carbon atoms, and preferably a C₁-C₁₈ alkyl or C₂-C₁₈ alkenyl radical;
R⁴ represents a divalent hydrocarbon radical, preferably a C₁-C₁₈ alkylene radical or a C₁-C₁₈ divalent alkyleneoxy radical;
Q⁻ is a halide ion;
r represents a mean statistical value from 2 to 20 and preferably from 2 to 8;
s represents a mean statistical value from 20 to 200 and preferably from 20 to 50.

12. Composition according to any one of Claims 1 to 7, **characterized in that** the polyoxyalkylenated silicone (ii)₂ is chosen from the compounds of the following formulae (IX), (X), (XI) and (XII) : 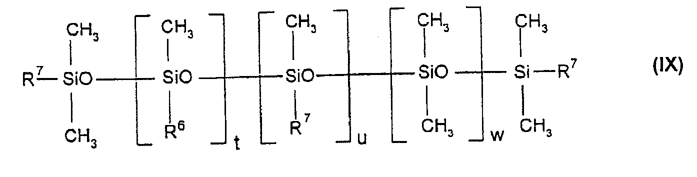 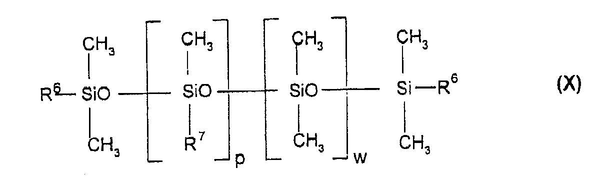 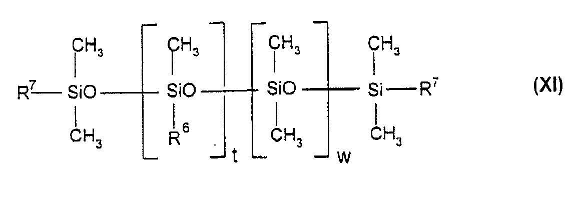 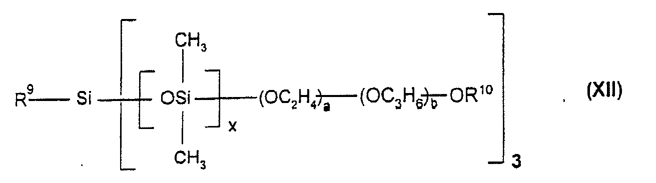 in which formulae (IX), (X), (XI) and (XII),
- R⁶, identical or different, represents a C₁-C₃₀, linear or branched, alkyl radical or a phenyl radical,
- R⁷, identical or different, represents a radical -C_{c}H_{2c}-O-(C₂H₄O)ₐ(C₃H₆O)_{b}-R⁸ or a radical -C_{c}H_{2c}-O- (C₄H₈O)ₐ-R⁸,
- R⁹, R¹⁰, which are identical or different, denote a C₁-C₁₂, linear or branched, alkyl radical, and preferably the methyl radical,
- R⁸, identical or different, is chosen from a hydrogen atom, a C₁-C₁₂, linear or branched, alkyl radical, a C₁-C₆, linear or branched, alkoxy radical, a C₂-C₃₀, linear or branched, acyl radical, a hydroxyl radical, an -SO₃M radical, a C₁-C₆ aminoalkoxy radical which is optionally substituted on the amine, a C₂-C₆ aminoacyl radical which is optionally substituted on the amine, a radical -NHCH₂CH₂COOM, a radical -N(CH₂CH₂COOM)₂, an aminoalkyl radical which is optionally substituted on the amine and on the alkyl chain, a C₂-C₃₀ carboxyacyl radical, a phosphono group which is optionally substituted with one or two substituted aminoalkyl radicals, a radical -CO(CH₂)_{d}COOM, -COCHR¹¹(CH₂)_{d}COOM, -NHCO(CH₂)_{d}OH, -NH₃Y, a phosphate group,
- M, identical or different, denotes a hydrogen atom, Na, K, Li, NH₄ or an organic amine,
- R¹¹ denotes a hydrogen atom or an -SO₃M radical,
- d varies from 1 to 10,
- u varies from 0 to 20,
- w varies from 0 to 500,
- t varies from 0 to 20,
- p varies from 1 to 50,
- a varies from 0 to 50,
- b varies from 0 to 50,
- the sum (a + b) is greater than or equal to 2,
- c varies from 0 to 4,
- x varies from 1 to 100,
- Y represents a monovalent inorganic or organic anion.

13. Composition according to Claim 12, **characterized in that** the silicone is chosen from those of formulae (X) or (XI) in which:
- c is equal to 2 or 3;
- R⁶ denotes methyl;
- R⁸ represents a methyl radical, a C₁₂-C₂₂ acyl radical or a -CO(CH₂)_{d}COOM radical;
- a varies from 2 to 25 and preferably from 2 to 15,
- b is equal to 0,
- w varies from 0 to 100,
- p varies from 1 to 20.

14. Composition according to any one of Claims 1 to 7, **characterized in that** the polyoxyalkylenated silicone (ii)₂ is chosen from the compounds of the following formula (XIII):
([Z (R¹²SiO)_{q}R¹³SiZO][(CₙH₂ₙO)ᵣ])ₛ **(XIII)**
in which,
- R¹² and R¹³, which are identical or different, represent a monovalent hydrocarbon radical, n is an integer ranging from 2 to 4, q is a number which is greater than or equal to 4, preferably between 4 and 200, r is a number which is greater than or equal to 4, preferably between 4 and 200, s is a number which is greater than or equal to 4, preferably between 4 and 1000, Z represents a divalent organic group which is linked to the adjacent silicon atom by a carbon-silicon bond and to a polyoxyalkylene block by an oxygen atom, the average molecular weight of each siloxane block being between 400 and 10,000, that of each polyoxyalkylene block being between 300 and 10,000, the siloxane blocks representing from 10 to 95% by weight of the block copolymer, and the number-average molecular weight of the block copolymer ranging from 2500 to 1,000,000, and preferably from 3000 to 200,000.

15. Composition according to any one of the preceding claims, **characterized in that** the silicone(s) (ii) represent from 0.01 to 20% by weight approximately of the total weight of the dyeing composition.

16. Composition according to Claim 15, **characterized in that** the silicone(s) represent from 0.1 to 10% by weight approximately of the total weight of the dyeing composition.

17. Composition according to any one of the preceding claims, **characterized in that** the appropriate dyeing medium (or carrier) consists of water or of a mixture of water and of at least one organic solvent.

18. Composition according to any one of the preceding claims, **characterized in that** it has a pH of between 2 and 11, and preferably between 5 and 10.

19. Composition according to any one of the preceding claims, **characterized in that** it is intended for oxidation dyeing and **in that** it contains one or more oxidation bases chosen from the para-phenylenediamines, the bis-phenylalkylenediamines, the para-aminophenols, the ortho-aminophenols and the heterocyclic bases.

20. Composition according to Claim 19, **characterized in that** the oxidation base(s) represent 0.0005 to 12% by weight of the total weight of the dyeing composition.

21. Composition according to Claim 20, **characterized in that** the oxidation base(s) represent 0.005 to 6% by weight of the total weight of the dyeing composition.

22. Composition according to any one of Claims 19 to 21, **characterized in that** it contains one or more couplers chosen from the the meta-phenylenediamines, the meta-aminophenols, the meta-diphenols and the heterocyclic couplers.

23. Composition according to Claim 22, **characterized in that** the coupler(s) represent from 0.0001 to 10% by weight of the total weight of the dyeing composition.

24. Composition according to Claim 23, **characterized in that** the coupler(s) represent from 0.005 to 5% by weight of the total weight of the dyeing composition.

25. Composition according to any one of the preceding claims, **characterized in that** it is intended for direct lightening dyeing or oxidation dyeing and **in that** it then contains at least one oxidizing agent.

26. Method of dyeing keratinous fibres and in particular human keratinous fibres such as hair, **characterized in that** at least one dyeing composition as defined in any one of Claims 1 to 25 is applied to the fibres for a sufficient time to develop the desired colour, after which they are rinsed, optionally washed with shampoo, rinsed again and dried.

27. Method of dyeing keratinous fibres and in particular human keratinous fibres such as hair, **characterized in that** at least one dyeing composition as defined in any one of Claims 1 to 25 is applied to the fibres for a sufficient time to develop the desired colour, with no final rinsing.

28. Method of dyeing keratinous fibres and in particular human keratinous fibres such as hair, **characterized in that** it comprises a preliminary stage consisting of storing in a separate form, on the one hand, a composition (A1) comprising, in an appropriate dyeing medium, at least one cationic direct dye (i) as defined in the preceding claims and at least one oxidation base and, on the other hand, a composition (B1) containing, in an appropriate dyeing medium, at least one oxidizing agent, and then mixing them at the time of use before applying this mixture to the keratinous fibres, the composition (A1) or the composition (B1) containing the silicone (ii) as defined in the preceding claims.

29. Method of dyeing keratinous fibres and in particular human keratinous fibres such as hair, **characterized in that** it comprises a preliminary stage consisting of storing in a separate form, on the one hand, a composition (A2) comprising, in an appropriate dyeing medium, at least one cationic direct dye (i) as defined in the preceding claims and, on the other hand, a composition (B2) containing, in an appropriate dyeing medium, at least one oxidizing agent, and then mixing them at the time of use before applying this mixture to the keratinous fibres, the composition (A2) or the composition (B2) containing the silicone (ii) as defined in the preceding claims.

30. Multi-compartment device or multi-compartment dyeing "kit", **characterized in that** a first compartment contains composition (A1) or (A2) as defined in Claim 28 or 29 and a second compartment contains composition (B1) or (B2) as defined in Claim 28 or 29.

## Patentansprüche

1. Zusammensetzung zum Färben von Keratinfasern und insbesondere menschlichen Keratinfasern, beispielsweise zum Färben des Haares, die in einem zum Färben geeigneten Medium enthält: (i) mindestens eine Verbindung, die unter den Verbindungen der folgenden Formeln (I) oder (III) ausgewählt ist:
(a) Verbindungen der folgenden Formel (I): 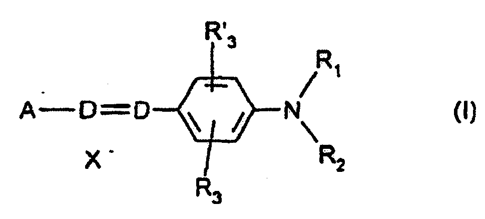 worin bedeuten:
D ein Stickstoffatom oder die Gruppe -CH,
R₁ und R₂, die gleich oder verschieden sind, ein Wasserstoffatom; eine C₁₋₄-Alkylgruppe, die mit einer Gruppe -CN, -OH oder -NH₂ substituiert sein kann, oder sie bilden mit einem Kohlenstoffatom des Benzolrings einen gegebenenfalls sauerstoff- oder stickstoff haltigen Heterocyclus, der mit einer oder mehreren C₁₋₄-Alkylgruppen substituiert sein kann; oder eine 4'-Aminophenylgruppe,
R₃ und R'₃, die identisch oder voneinander verschieden sind, ein Wasserstoffatom, ein Halogenatom, das unter Chlor, Brom, Iod und Fluor ausgewählt ist, Cyano, C₁₋₄-Alkyl, C₁₋₄-Alkoxy oder Acetyloxy,
X- ein Anion, das vorzugsweise unter Chlorid, Methylsulfat und Acetat ausgewählt ist,
A eine Gruppe, die unter den folgenden Strukturen A1 bis A 18 ausgewählt ist: 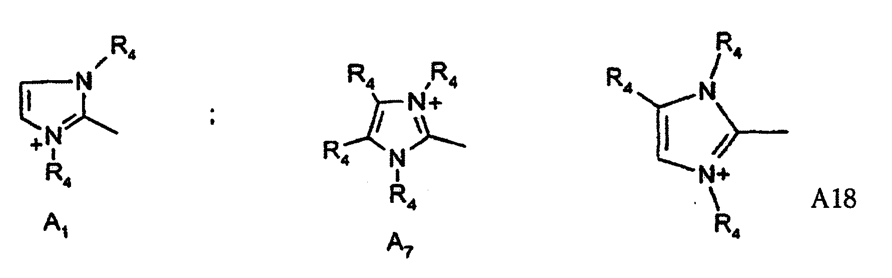 worin die Gruppe R₄ eine C₁₋₄-Alkylgruppe bedeutet, die mit einer Hydroxygruppe substituiert sein kann, und R₅ eine C₁₋₄-Alkoxygruppe ist, mit der Maßgabe, dass die Gruppen R₁ und R₂ nicht gleichzeitig ein Wasserstoffatom bedeuten, wenn D die Gruppe -CH ist, die Gruppe A A₄ oder A₁₃ bedeutet und R₃ von Alkoxy verschieden ist;
(b) Verbindungen der folgenden Formel (III): 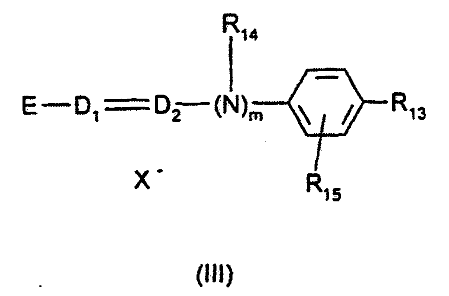 worin bedeuten:
R₁₃ ein Wasserstoffatom, eine C₁₋₄-Alkoxygruppe, ein Halogenatom, wie Brom, Chlor, Iod oder Fluor, oder eine Aminogruppe, R₁₄ ein Wasserstoffatom oder eine C₁₋₄-Alkylgruppe oder R₁₄ bildet mit einem Kohlenstoffatom des Benzolrings einen Heterocyclus, der gegebenenfalls sauerstoffhaltig und/oder mit einer oder mehreren C₁₋₄-Alkylgruppen substituiert ist;
R₁₅ ein Wasserstoffatom oder ein Halogenatom, wie Brom, Chlor, Iod oder Fluor,
D₁ und D₂, die gleich oder verschieden sind, ein Stickstoffatom oder die Gruppe -CH,
m = 0 oder 1,
mit der Maßgabe, dass die Gruppen D₁ und D₂ gleichzeitig -CH bedeuten und m = 0, wenn R₁₃ eine unsubstituierte Aminogruppe ist,
X⁻ ein Anion, das vorzugsweise unter Chlorid, Methylsulfat und Acetat ausgewählt ist,
E eine Gruppe, die unter den folgenden Strukturen E1 bis E8 ausgewählt ist: 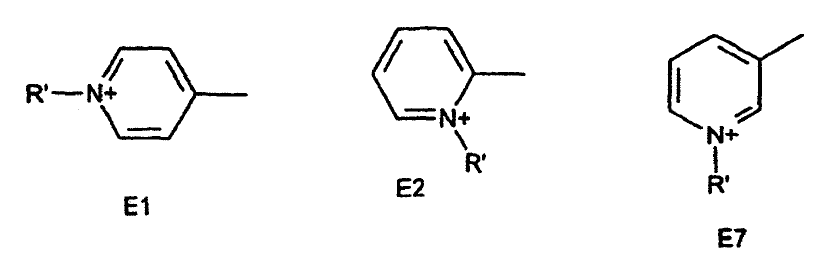 wobei R' eine C₁₋₄-Alkylgruppe bedeutet;
wobei die Zusammensetzung **dadurch gekennzeichnet ist, dass** sie ferner ein Silicon enthält, das unter den folgenden Verbindungen ausgewählt ist:
(ii)₁ - aminierten Siliconen;
(ii)₂ - polyalkoxylierten Siliconen;
(ii)₃ - Silicongummis und Siliconharzen.

2. Zusammensetzung nach Anspruch 1, **dadurch gekennzeichnet, dass** die kationischen Direktfarbstoffe der Formel (I) unter den Verbindungen der folgenden Strukturen (I1) bis (I54) ausgewählt sind:

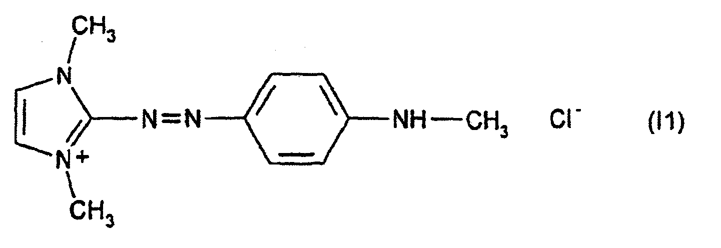

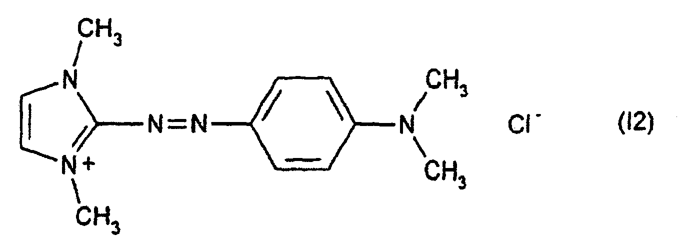

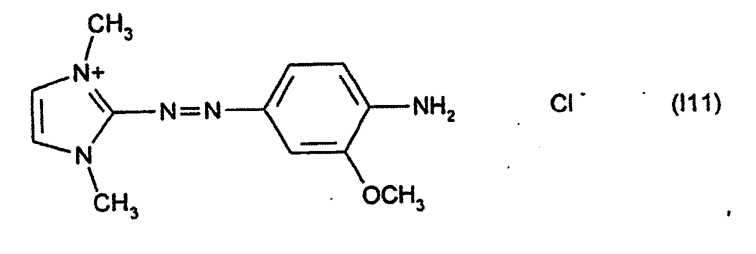

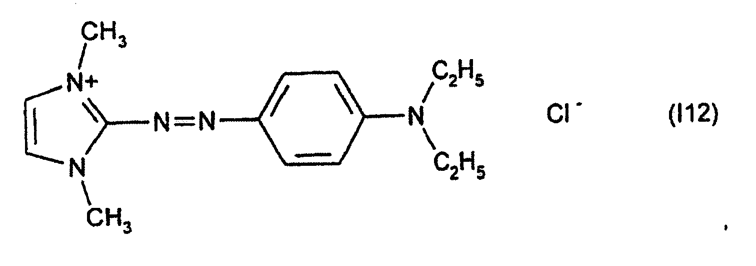

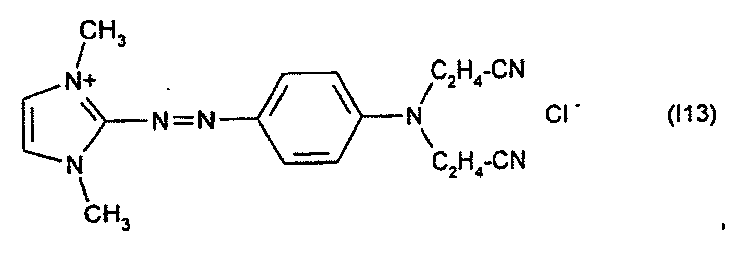

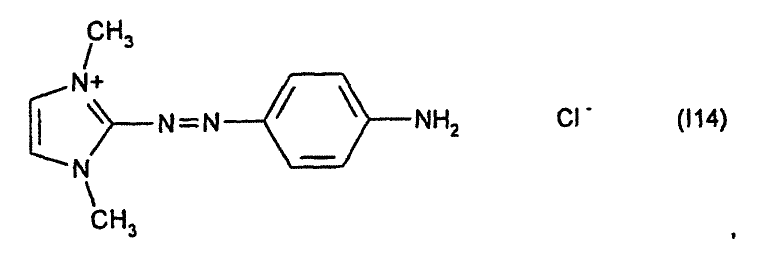

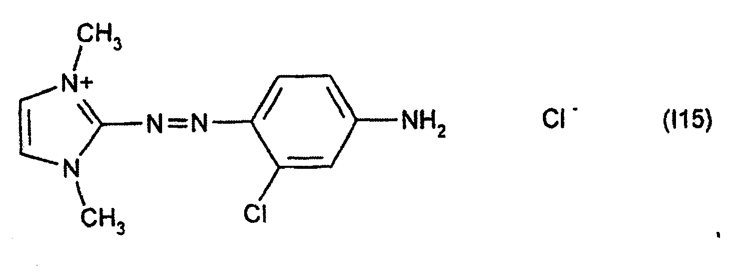

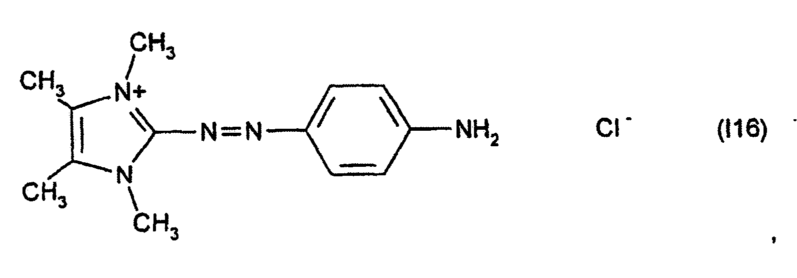

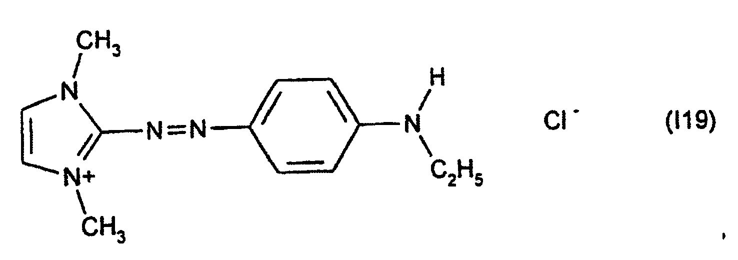

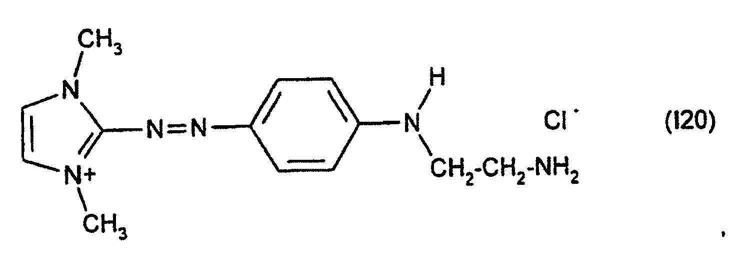

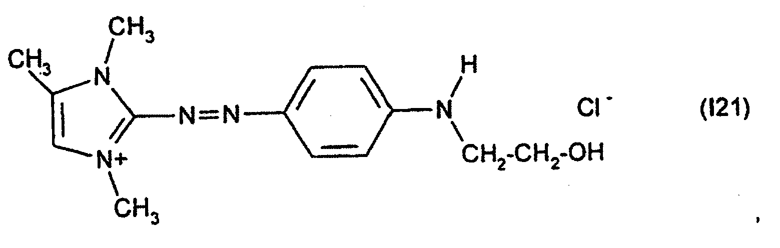

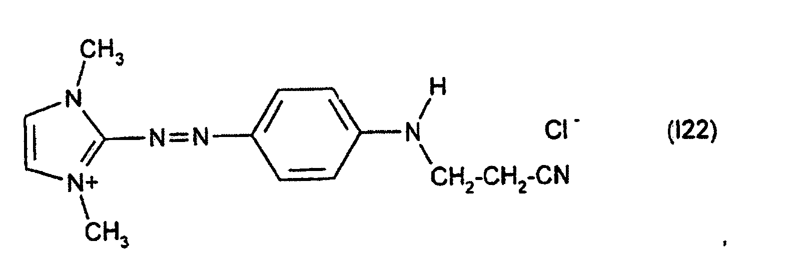

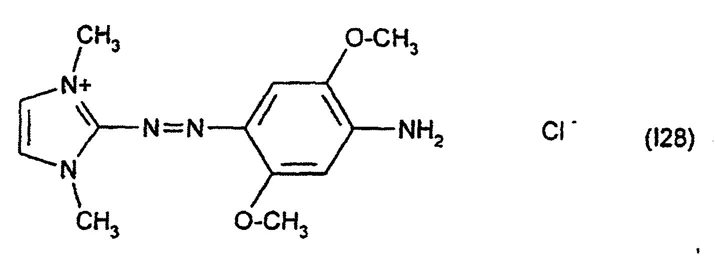

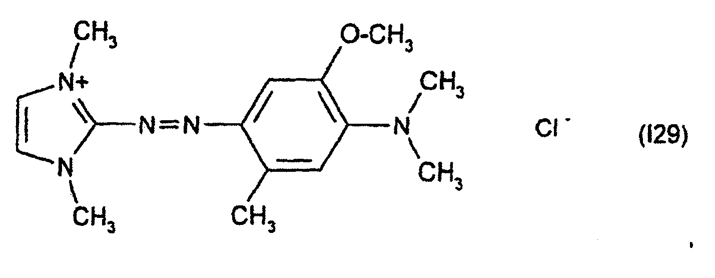

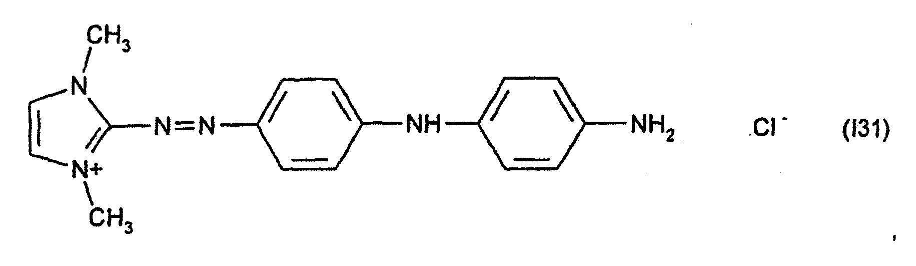

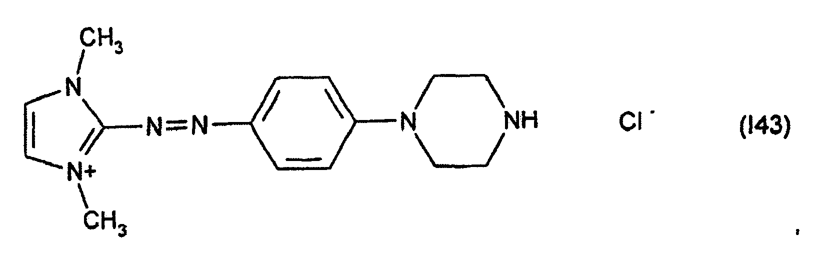

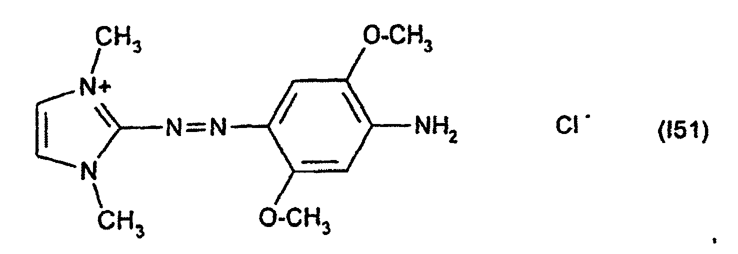

3. Zusammensetzung nach Anspruch 2, **dadurch gekennzeichnet, dass** die kationischen Direktfarbstoffe den Strukturen (I1), (I2), (I14) und (I31) entsprechen.

4. Zusammensetzung nach Anspruch 1, **dadurch gekennzeichnet, dass** die kationischen Direktfarbstoffe der Formel (III) unter den Verbindungen der folgenden Strukturen (III1) bis (III18) ausgewählt sind:

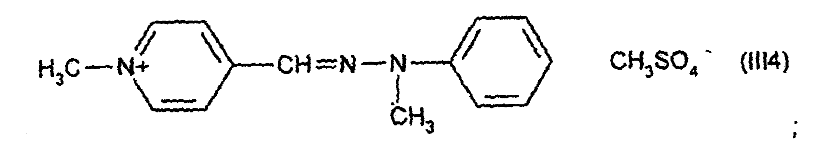

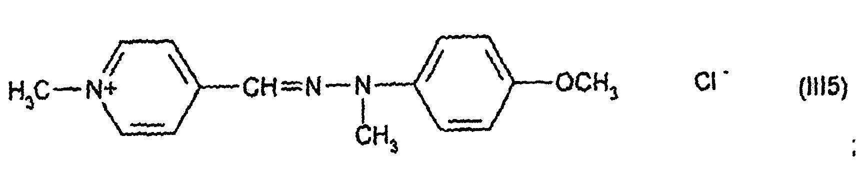

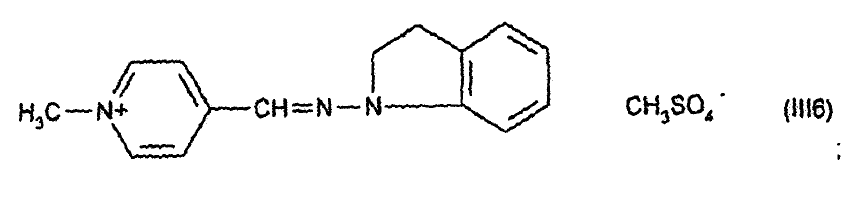

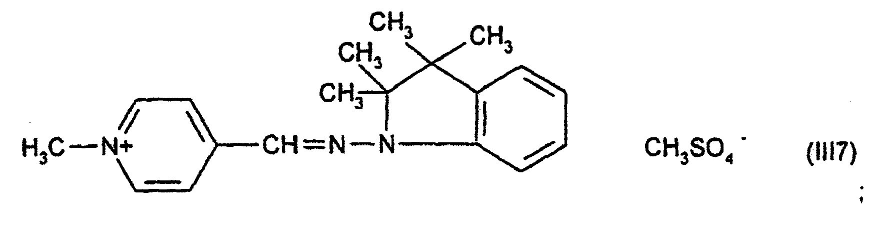

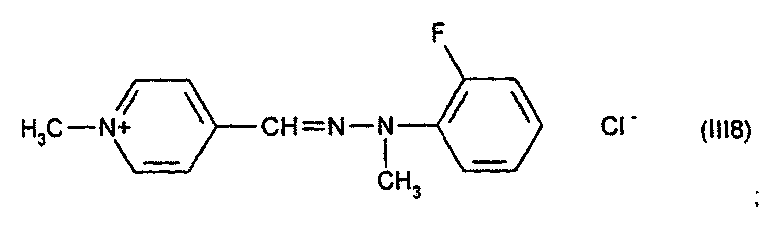

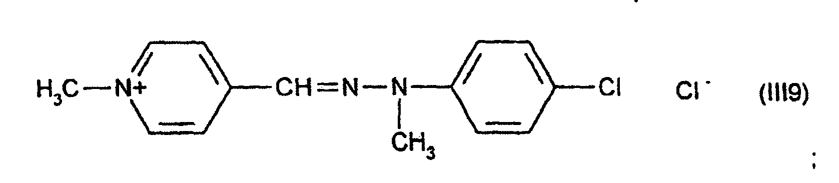

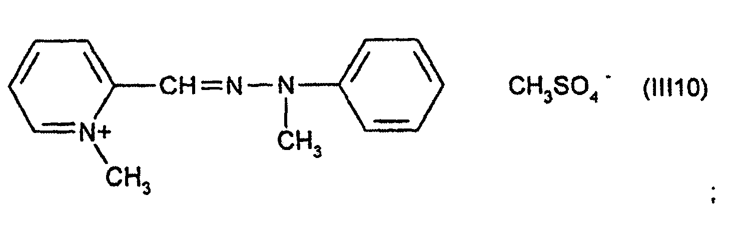

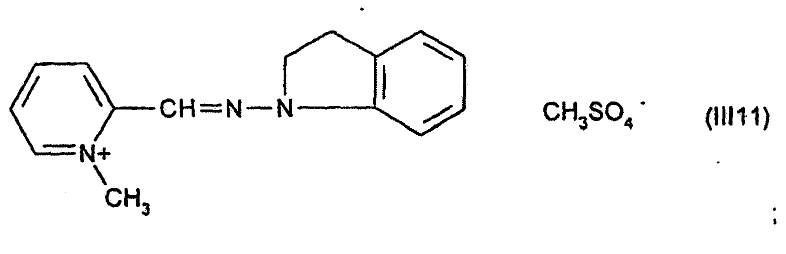

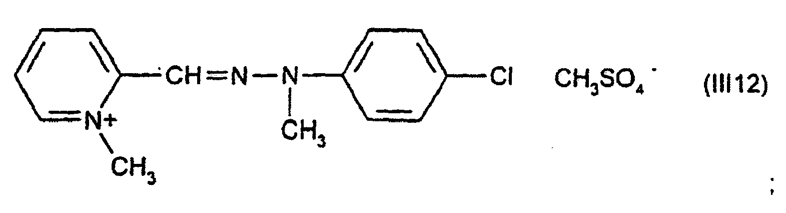

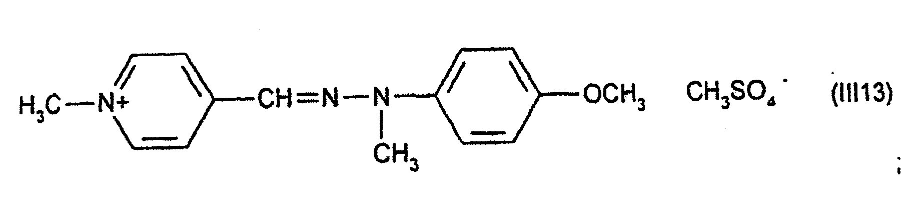

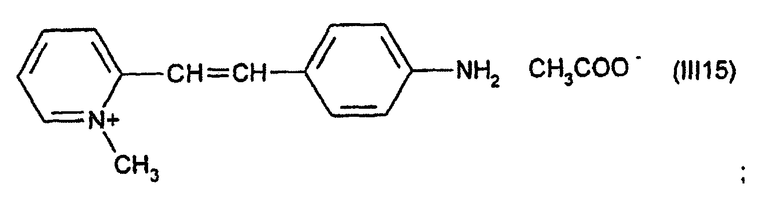

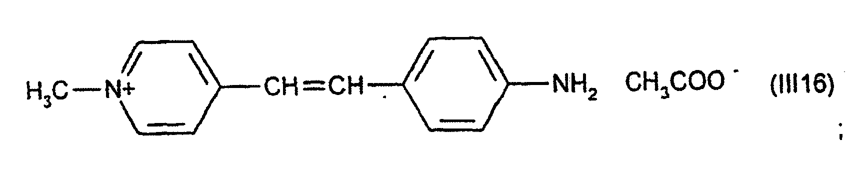

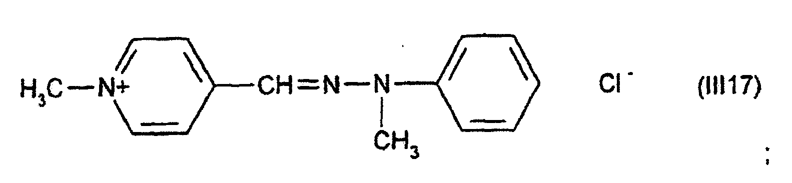

5. Zusammensetzung nach Anspruch 4, **dadurch gekennzeichnet, dass** die kationischen Direktfarbstoffe der Formel (III) den Strukturen (III4), (III5) und (III13) entsprechen.

6. Zusammensetzung nach einem der vorhergehenden Ansprüche, **dadurch gekennzeichnet, dass** der oder die kationische(n) Direktfarbstoff(e) der Formeln (I) oder (III) 0,001 bis 10 Gew.-% des Gesamtgewichts der Zusammensetzung ausmachen.

7. Zusammensetzung nach Anspruch 6, **dadurch gekennzeichnet, dass** der oder die kationische(n) Direktfarbstoff(e) der Formeln (I) oder (III) 0,005 bis 5 Gew.-% des Gesamtgewichts der Zusammensetzung ausmachen.

8. Zusammensetzung nach einem der vorhergehenden Ansprüche, **dadurch gekennzeichnet, dass** das aminierte Silicon (ii)₁ unter den Verbindungen der folgenden Formel (V) ausgewählt ist: 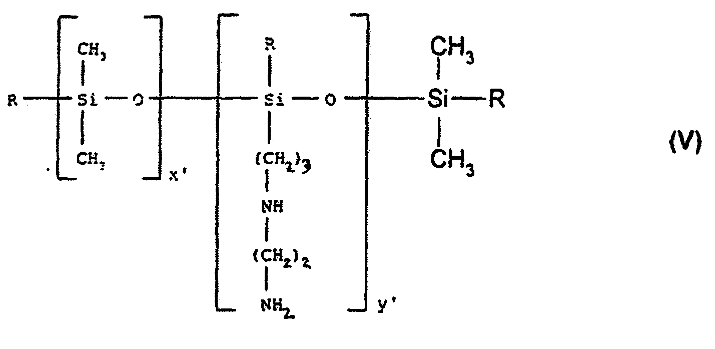
wobei R die Gruppe CH₃ oder OH bedeutet und
x' und y' ganze Zahlen bedeuten, die von der Molmasse abhängen.

9. Zusammensetzung nach einem der Ansprüche 1 bis 7, **dadurch gekennzeichnet, dass** das aminierte Silicon (ii)₁ unter den Verbindungen der folgenden Formel (VI) ausgewählt ist:
(R¹)ₐ (T)₃₋ₐ -Si[OSi(T)₂]ₙ -[OSi(T)_{b}(R¹)_{2-b}]ₘ -OSi(T)₃₋ₐ -(R¹)ₐ **(VI)**
worin bedeuten:
- T Wasserstoff, Phenyl, OH oder C₁₋₈-Alkyl, vorzugsweise Methyl,
- a 0 oder eine ganze Zahl von 1 bis 3 und vorzugsweise 0,
- b 0 oder 1 und insbesondere 1,
- m und n Zahlen, die so ausgewählt sind, dass die Summe (n + m) insbesondere im Bereich von 1 bis 2000 und besonders 50 bis 150 liegen kann, wobei n eine Zahl von 0 bis 1999 und insbesondere 49 bis 149 und m eine Zahl von 1 bis 2000 und insbesondere 1 bis 10 bedeuten kann,
- die Gruppe R¹ eine einwertige Gruppe der Formel -C_{q}H_{2q}L, worin q eine Zahl von 2 bis 8 ist und L eine gegebenenfalls quaternisierte, aminierte Gruppe bedeutet, welche unter den folgenden Gruppen ausgewählt ist:
-N(R²)-CH₂-CH₂-N(R²)₂; -N(R²)₂; -N^{⊕}(R²)₃Q⁻; -N^{⊕}(R²)(H)₂Q⁻;
-N^{⊕}(R²)₂HQ⁻; -N(R²)-CH₂-CH₂-N^{⊕}(R²)(H)₂ Q-,
worin die Gruppen R² Wasserstoff, Phenyl, Benzyl oder eine einwertige gesättigte Kohlenwasserstoffgruppe, vorzugsweise eine Alkylgruppe mit 1 bis 20 Kohlenstoffatomen, bedeuten und Q- ein Halogenid bedeutet.

10. Zusammensetzung nach Anspruch 9, **dadurch gekennzeichnet, dass** das aminierte Silicon unter den Verbindungen der folgenden Formel (VII) ausgewählt ist: 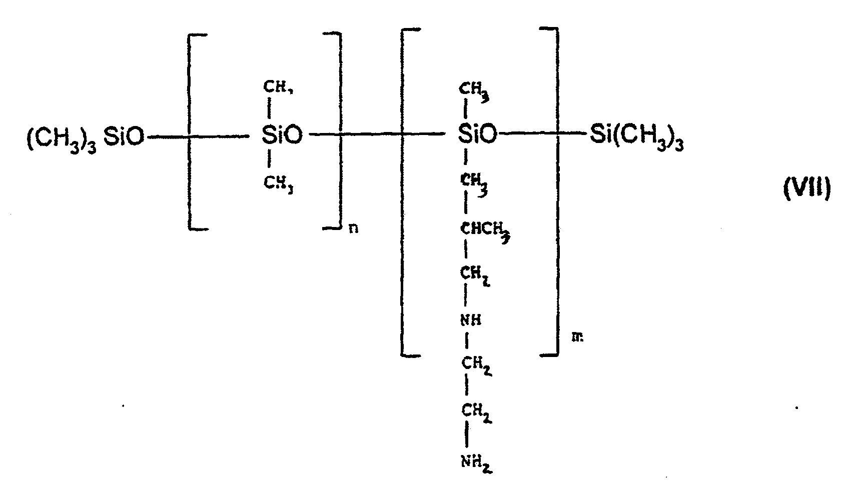 worin n und m die oben für Formel (VI) angegebenen Bedeutungen aufweisen.

11. Zusammensetzung nach einem der Ansprüche 1 bis 7, **dadurch gekennzeichnet, dass** das aminierte Silicon (ii)₁ unter den Verbindungen der folgenden Formel (VIII) ausgewählt ist: 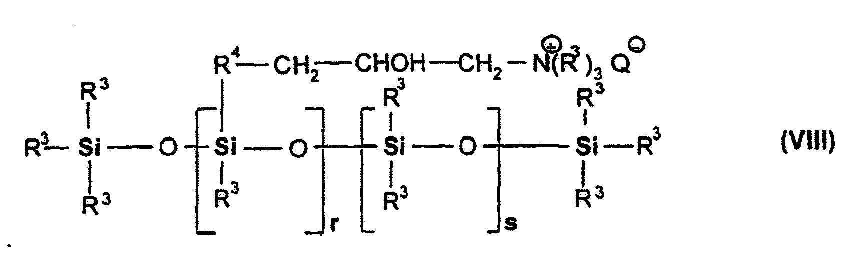 worin bedeuten:
R³ eine einwertige Kohlenwasserstoffgruppe mit 1 bis 18 Kohlenstoffatomen und vorzugsweise eine C₁₋₁₈-Alkylgruppe oder eine C₂₋₁₈-Alkenylgruppe,
R⁴ eine zweiwertige Kohlenwasserstoffgruppe und vorzugsweise eine Alkylengruppe mit 1 bis 18 Kohlenstoffatomen oder eine zweiwertige Alkylenoxygruppe mit 1 bis 18 Kohlenstoffatomen,
Q- ein Halogenid,
r einen statistischen Mittelwert von 2 bis 20 und vorzugsweise 2 bis 8, und
s einen statistischen Mittelwert von 20 bis 200 und vorzugsweise 20 bis 50.

12. Zusammensetzung nach einem der Ansprüche 1 bis 7, **dadurch gekennzeichnet, dass** das polyalkoxylierte Silicon (ii)₂ unter den Verbindungen der folgenden Formeln (IX), (X), (XI) und (XII) ausgewählt ist: 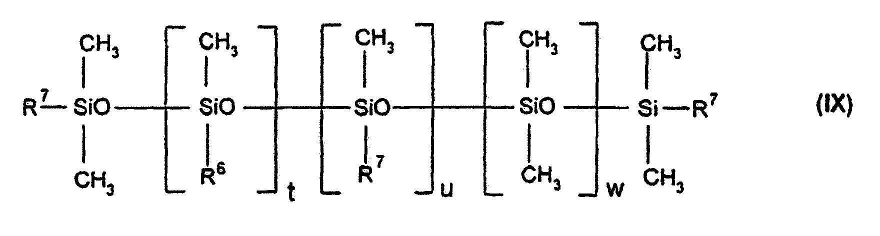 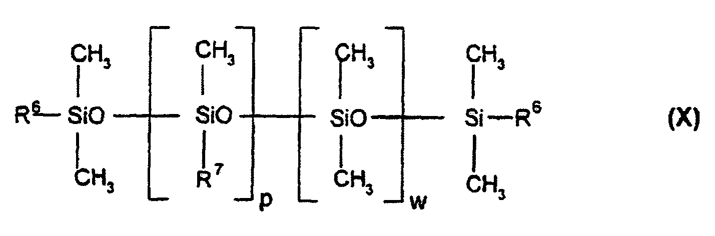 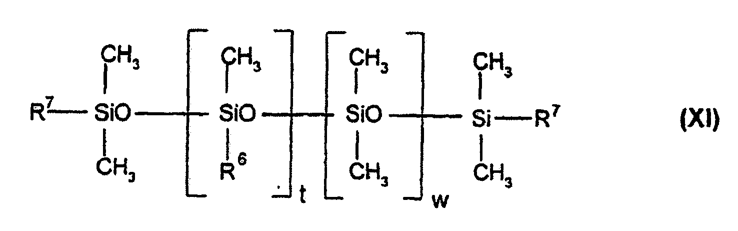 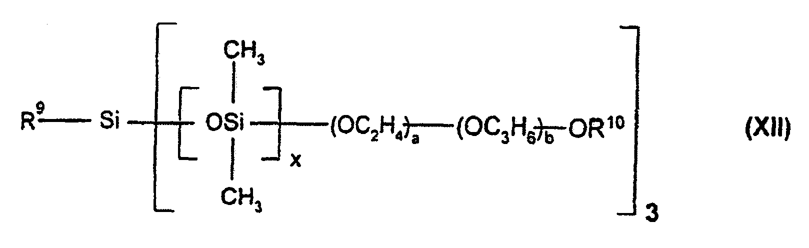 wobei in den Formeln (IX), (X), (XI) und (XII):
- die Gruppen R⁶, die identisch oder voneinander verschieden sind, eine geradkettige oder verzweigte C₁₋₃₀-Alkylgruppe oder Phenyl bedeuten,
- die Gruppen R⁷, die identisch oder voneinander verschieden sind, eine Gruppe -C_{c}H_{2c}-O-(C₂H₄O)ₐ(C₃H₆O)_{b}-R⁸ oder eine Gruppe -C_{c}H_{2c}-O-(C₄H₈O)ₐ-R⁸ bedeuten,
- die Gruppen R⁹ und R¹⁰, die identisch oder voneinander verschieden sind, eine geradkettige oder verzweigte C₂₋₁₂-Alkylgruppe und vorzugsweise Methyl bedeuten,
- die Gruppen R⁸, die identisch oder voneinander verschieden sind, unter einem Wasserstoffatom, einer geradkettigen oder verzweigten Alkylgruppe mit 1 bis 12 Kohlenstoffatomen, einer geradkettigen oder verzweigten Alkoxygruppe mit 1 bis 6 Kohlenstoffatomen, einer geradkettigen oder verzweigten Acylgruppe mit 2 bis 30 Kohlenstoffatomen, Hydroxy, -SO₃M, einer gegebenenfalls an der Aminogruppe substituierten C₁₋₆-Aminoalkoxygruppe, einer gegebenenfalls an der Aminogruppe substituierten C₂₋₆-Aminoacylgruppe, -NHCH₂CH₂COOM, -N(CH₂CH₂COOM)₂, einer gegebenenfalls an der Aminogruppe und an der Alkylgruppe substituierten Aminoalkylgruppe, einer C₂₋₃₀-Carboxyacylgruppe, einer Phosphonoruppe, die gegebenenfalls mit einer oder zwei substituierten Aminoalkylgruppen substituiert ist, -CO(CH₂)_{d}COOM, -COCHR¹¹(CH₂)_{d}COOM, -NHCO(CH₂)_{d}OH, -NH₃Y und einer Phosphatgruppe ausgewählt sind,
- die Gruppen M, die gleich oder verschieden sind, Wasserstoff, Na, K, Li, NH₄ oder ein organisches Amin bedeuten,
- R¹¹ Wasserstoff oder SO₃M ist,
- d im Bereich von 1 bis 10 liegt,
- u im Bereich von 0 bis 20 liegt,
- w im Bereich von 0 bis 500 liegt,
- t im Bereich von 0 bis 20 liegt,
- p im Bereich von 1 bis 50 liegt,
- a im Bereich von 0 bis 50 liegt,
- b im Bereich von 0 bis 50 liegt,
- a + b mindestens 2 bedeutet,
- c im Bereich von 0 bis 4 liegt,
- x im Bereich von 1 bis 100 liegt, und
- Y ein anorganisches oder organisches einwertiges Anion bedeutet.

13. Zusammensetzung nach Anspruch 12, **dadurch gekennzeichnet, dass** das Silicon unter den Siliconen der Formeln (X) oder (XI) ausgewählt ist, worin bedeuten:
- c bedeutet 2 oder 3,
- R⁶ ist Methyl,
- R⁸ bedeutet Methyl, C₁₂₋₂₂-Acyl oder -CO(CH₂)_{d}COOM,
- a liegt im Bereich von 2 bis 25 und vorzugsweise 2 bis 15,
- b ist 0,
- w liegt im Bereich von 0 bis 100, und
- p liegt im Bereich von 1 bis 20.

14. Zusammensetzung nach einem der Ansprüche 1 bis 7, **dadurch gekennzeichnet, dass** das polyalkoxylierte Silicon (ii)₂ unter den Verbindungen der folgenden Formel (XIII) ausgewählt ist:
([Z(R¹²SiO)_{q}R¹³SiZO] [(CₙH₂ₙO)ᵣ])ₛ **(XIII)**
worin bedeuten:
die Gruppen R¹² und R¹³, die identisch oder voneinander verschieden sind, eine einwertige Kohlenwasserstoffgruppe, n eine ganze Zahl von 2 bis 4, q mindestens 4, vorzugsweise eine Zahl im Bereich von 4 bis 200, r mindestens 4, vorzugsweise eine Zahl im Bereich von 4 bis 200, s mindestens 4, vorzugsweise eine Zahl im Bereich von 4 bis 1000, Z eine zweiwertige organische Gruppe, die über eine Kohlenstoff-Silicium-Bindung an das angrenzende Siliciumatom und über ein Sauerstoffatom an einen Polyoxyalkylenblock gebunden ist; wobei die mittlere Molmasse jedes Siloxanblocks im Bereich von 400 bis 10 000 und die mittlere Molmasse jedes Polyoxyalkylenblocks im Bereich von 300 bis 10 000 liegt, die Siloxanblöcke 10 bis 95 Gew.-% des Blockcopolymers ausmachen und das zahlenmittlere Molekulargewicht des Blockcopolymers im Bereich von 2 500 bis 1 000 000 und vorzugsweise 3 000 bis 200 000 liegt.

15. Zusammensetzung nach einem der vorhergehenden Ansprüche, **dadurch gekennzeichnet, dass** das oder die Silicon(e) (ii) etwa 0,01 bis 20 Gew.-% des Gesamtgewichts der Farbmittelzusammensetzung ausmachen.

16. Zusammensetzung nach Anspruch 15, **dadurch gekennzeichnet, dass** das oder die Silicon(e) (ii) etwa 0,1 bis 10 Gew.-% des Gesamtgewichts der Farbmittelzusammensetzung ausmachen.

17. Zusammensetzung nach einem der vorhergehenden Ansprüche, **dadurch gekennzeichnet, dass** das zum Färben geeignete Medium (oder der Träger) aus Wasser oder einem Gemisch von Wasser und mindestens einem organischen Lösungsmittel besteht.

18. Zusammensetzung nach einem der vorhergehenden Ansprüche, **dadurch gekennzeichnet, dass** sie einen pH-Wert im Bereich von 2 bis 11 und vorzugsweise 5 bis 10 aufweist.

19. Zusammensetzung nach einem der vorhergehenden Ansprüche, **dadurch gekennzeichnet, dass** sie zum oxidativen Färben vorgesehen ist und eine oder mehrere Oxidationsbasen enthält, die unter den p-Phenylendiaminen, Bisphenylalkylendiaminen, p-Aminophenolen, o-Aminophenolen und heterocyclischen Basen ausgewählt sind.

20. Zusammensetzung nach Anspruch 19, **dadurch gekennzeichnet, dass** die Oxidationsbase(n) 0,0005 bis 12 Gew.-% des Gesamtgewichts der Farbmittelzusammensetzung ausmachen.

21. Zusammensetzung nach Anspruch 20, **dadurch gekennzeichnet, dass** die Oxidationsbase(n) 0,005 bis 6 Gew.-% des Gesamtgewichts der Farbmittelzusammensetzung ausmachen.

22. Zusammensetzung nach einem der Ansprüche 19 bis 21, **dadurch gekennzeichnet, dass** sie einen oder mehrere Kuppler enthält, die unter den m-Phenylendiaminen, m-Aminophenolen, m-Dihydroxybenzolen und heterocyclischen Kupplern ausgewählt sind.

23. Zusammensetzung nach Anspruch 22, **dadurch gekennzeichnet, dass** der oder die Kuppler 0,0001 bis 10 Gew.-% des Gesamtgewichts der Farbmittelzusammensetzung ausmachen.

24. Zusammensetzung nach Anspruch 23, **dadurch gekennzeichnet, dass** der oder die Kuppler 0,005 bis 5 Gew.-% des Gesamtgewichts der Farbmittelzusammensetzung ausmachen.

25. Zusammensetzung nach einem der vorhergehenden Ansprüche, **dadurch gekennzeichnet, dass** sie für die aufhellende Direktfärbung oder zum oxidativen Färben vorgesehen ist und mindestens ein Oxidationsmittel enthält.

26. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 25 während einer Zeitspanne, die zur Bildung der gewünschten Färbung ausreichend ist, aufgebracht wird und dann gespült, gegebenenfalls mit Haarwaschmittel gewaschen, nochmals gespült und getrocknet wird.

27. Verfahren zum Färben von Keratinfasern und insbesondere menschlichen Keratinfasern, wie Haaren, **dadurch gekennzeichnet, dass** auf die Fasern mindestens eine Farbmittelzusammensetzung nach einem der Ansprüche 1 bis 25 während einer Zeitspanne, die zur Bildung der gewünschten Färbung ausreichend ist, aufgebracht wird, ohne dass nochmals gespült wird.

28. Verfahren zum Färben von Keratinfasern und insbesondere menschlichen Keratinfasern, wie Haaren, **dadurch gekennzeichnet, dass** es einen vorbereitenden Schritt umfasst, der darin besteht, einerseits eine Zusammensetzung (A1), die in einem zum Färben geeigneten Medium mindestens einen in den vorhergehenden Ansprüchen definierten Direktfarbstoff (i) und mindestens eine Oxidationsbase enthält, und andererseits eine Zusammensetzung (B1) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A1) oder die Zusammensetzung (B1) das in den vorhergehenden Ansprüchen definierte Silicon (ii) enthält.

29. Verfahren zum Färben von Keratinfasern und insbesondere menschlichen Keratinfasern, wie Haaren, **dadurch gekennzeichnet, dass** es einen vorbereitenden Schritt umfasst, der darin besteht, einerseits eine Zusammensetzung (A2), die in einem zum Färben geeigneten Medium mindestens einen in den vorhergehenden Ansprüchen definierten kationischen Direktfarbstoff (i) enthält, und andererseits eine Zusammensetzung (B2) getrennt voneinander aufzubewahren, die in einem zum Färben geeigneten Medium mindestens ein Oxidationsmittel enthält, und diese bei der Anwendung zu vermischen, bevor das Gemisch auf die Fasern aufgebracht wird, wobei die Zusammensetzung (A2) oder die Zusammensetzung (B2) das in den vorhergehenden Ansprüchen definierte Silicon (ii) enthält.

30. Vorrichtung mit mehreren Abteilungen oder "Kit" zum Färben mit mehreren Abteilungen, **dadurch gekennzeichnet, dass** eine erste Abteilung die Zusammensetzung (A1) oder (A2) nach Anspruch 28 oder 29 und eine andere Abteilung die Zusammensetzung (B1) oder (B2) nach Anspruch 28 oder 29 enthält.
